# EUROPEAN PATENT APPLICATION

(11) **EP 4 846 930 A1**
(43) Date of publication of application: **02.12.2026**
(21) Application number: 26182132.6
(22) Date of filing: 29.05.2026
(51) Int. Cl.: A61B 5/00, A61B 5/372

(54) **SYSTEMS AND METHODS FOR INTEGRATION OF ELECTROPHYSIOLOGICAL DATA WITH A VISUAL REPRESENTATION OF ASSOCIATED ELECTRODES AND CONTACTS OF INTEREST IN A THREE-DIMENSIONAL BRAIN IMAGE**

(30) Priority: 30.05.2025 US 202563814681 P
(71) Applicant: Cadwell Laboratories, Inc., Richland, WA 99336 (US); Cadwell, John A., Richland, Washington 99352 (US)
(72) Inventor: Holub, Michal, 730707 Singapore (SG); Bell, Robert, Coeur d'Alene, 83815 (US); Barcus, Scott, Kennewick, 99337 (US); Cadwell, John A., Richland, Washington 99352 (US)
(74) Representative: Murgitroyd & Company

(57) **Abstract**

A method of enabling a clinician to visualize, review, and analyze a patient's EEG data and a visual representation of corresponding electrodes and contacts includes generating a unified viewing environment, wherein the unified viewing environment displays, simultaneously, EEG traces in a first view area and the visual representation of corresponding electrodes and contacts within a patient-specific 3D brain images in a second view area of the unified viewing environment. The method includes automatically associating the visual representation of each of the electrodes and contacts with an EEG trace, enabling the clinician to select contacts of interest (COI) within and/or across electrodes based on anatomical/functional regions and/or neural connectivity data of the patient's brain, automatically generating at least one group of one or more COI montages, and performing a plurality of EEG analyses based on the at least one group of one or more COI montages.

## Description

### CROSS-REFERENCE

The present application relies on, for priority, United States Patent Provisional Application Number 63/814,681, titled "Systems and Methods for Integration of Electrophysiological Data with a Visual Representation of Associated Electrodes and Contacts of Interest in a Three-Dimensional Brain Image" and filed on May 30, 2025, which is herein incorporated by reference in its entirety.

### FIELD

The present specification is related generally to the field of diagnostic systems. More specifically, the present specification is related to the integration of electrophysiological data with a visual representation of correlated electrodes and contacts in a patient-specific three-dimensional brain image to support a plurality of analytical and visual functionalities.

### BACKGROUND

An EEG trace is generated by measuring the voltage difference between an active electrode (i.e. a single active contact) and a reference electrode (which may have one or multiple contacts). The arrangement of these electrode pairs across the scalp is known as a montage and determines how brain activity appears in the resulting waveform pattern. When using standard electrode placements such as the 10-20 system, clinicians can intuitively map EEG signals to specific brain regions. However, arbitrary electrode arrangements and placements without anatomical correlation make it difficult to localize neural activity origins, interpret signal patterns meaningfully, and compare findings across different recordings.

Intracranial EEG initially involves placing individual electrodes directly on the brain's surface and recording EEG activity with the brain exposed - a method known as Electrocorticography (ECoG). A novel method, utilizing strip and grid electrodes, was developed to enable extended intracranial EEG recordings, usually for the localization of the epileptogenic focus. This method combines multiple contacts into a single electrode, either in a two-dimensional matrix (grid) or a single line (strip). The strips and grids are positioned directly on the brain surface. The brain is then covered up again with the electrodes remaining inside the skull, whereby EEG activity is recorded over a time period on the order of multiple days. Since the strip and grid electrodes are laid out in an organized fashion, it is relatively straightforward for clinicians to understand the anatomical location of each electrode contact with montages that either go horizontally or vertically. ECoG has two primary drawbacks: it is quite invasive, which may result in significant long-term effects on the patient, and it primarily yields information from the brain's surface, offering little to no insight into the deeper brain structures.

Consequently, a technique known as Stereo EEG (SEEG) was developed, which involves drilling small openings in the skull to insert long, thin electrodes with multiple contacts deep into the brain. This less invasive method permits the use of a greater number of electrodes and contacts, and it allows placing contacts throughout a three-dimensional volume. The primary challenge that arises from this technique, however, is that it complicates the understanding of the spatial arrangement of the various electrode contacts within the brain and their positional relationships with one another. The only well-defined relationships among contacts are along the axis of each electrode, the number of possible relations across electrodes is high and is usually not shown, and the increased number of contacts and corresponding EEG data must be analyzed and reviewed. This is the primary reason that, despite the distinct anatomical organization of SEEG electrodes, the same EEG montage methodology has been retained from the extracranial and strip/grid techniques. This constraint limits the clinical interpretation to a very small subset of the extensive EEG information that is available.

Established anatomical standards for electrode placement-such as the 10-20, 10-10, and 10-05 systems-precisely define and localize specific groups of electrodes (for example, C3, Cz, and C4), anatomically, and form the basis of extracranial EEG procedures. To allow for consistent analysis of brain activity using EEG, several standard montages, such as Double Banana, Transversal, and Laplacian have been defined using these electrode positioning standards. A montage in electroencephalography (EEG) refers to the specific arrangement and selection of electrode pairs (called derivations or channels) that are used to record and display brain activity on an EEG tracing. Each montage determines which electrodes are connected to each amplifier input and how the resulting signals are organized for interpretation. There are several common types of montages: a) for a bipolar montage, each channel records the voltage difference between two adjacent electrodes, often arranged in chains across the scalp; b) for a referential (monopolar montage), each channel records the difference between a scalp electrode and a common reference electrode, such as the earlobe or mastoid; c) for an average reference montage, the average signal from all electrodes is used as a reference for each channel; and d) a Laplacian montage uses a weighted average of surrounding electrodes as the reference, emphasizing local activity. Montages are essential for localizing and interpreting EEG signals, as the choice and arrangement of channels can highlight or obscure different patterns of brain activity. The selection of a montage is based on the clinical or research objective and can be adjusted to optimize visualization of specific EEG features.

Clinicians have been able to learn and recognize various EEG patterns that correlate to various clinical brain states and pathologies by using these standard EEG electrode positioning and montaging modalities. It is generally believed that a clinician must actively read EEG traces for a year before becoming proficient enough to provide clinical analyses. In most cases, montages are referential (all data is derived from one or more specific electrodes compared to a common reference electrode) or bipolar (examining data between two adjacent electrodes). At present, the analysis of intracranial electrical data is constrained by time and effort, limiting it to either examining the differences in electrical activity between two adjacent contacts of the same electrode (bipolar montage) or comparing each electrode contact to a distinct common reference contact (referential or average reference montages). This restriction hinders clinicians from accessing critical clinical insights.

Accordingly, there is need for systems and methods that enable rapid creation of montages and EEG trace data based on contacts of interest for an interactive and dynamic exploration of a patient's electrophysiological profile. There is also a need for the integration of EEG signals/traces with anatomical images in a unified viewing environment that supports a clinician by optimizing an understanding of the actual source of electrical activity and its spread through the neural network of the patient's brain.

### SUMMARY

The following embodiments and aspects thereof are described and illustrated in conjunction with systems, tools and methods, which are meant to be exemplary and illustrative, and not limiting in scope. The present application discloses numerous embodiments.

The present specification discloses a computer-implemented method of enabling a user to generate one or more montages using a computing device, wherein the computing device comprises at least one processor configured to execute programmatic instructions and a non-transient memory adapted to store the programmatic instructions, the method comprising: generating a graphical user interface, wherein the graphical user interface comprises at least a first display area, a second display area and a third display area, wherein the first display area, the second display area and the third display area are concurrently displayed and visually separated from each other; acquiring, using the computing device, an image of a patient's brain comprising data indicative of a visual representation of one or more electrodes placed in the patient's brain, wherein each of the one or more electrodes has associated one or more contacts therewith, and wherein the three-dimensional image is aligned with a three-dimensional coordinate system; acquiring, using the computing device, EEG data of the patient, wherein the EEG data comprises a first plurality of EEG traces; displaying, simultaneously, one or more of the first plurality of EEG traces in the first display area and the image in the second display area; automatically visually associating the visual representation of each of the one or more electrodes and associated one or more contacts in the image with a corresponding one of the one or more first plurality of EEG traces; receiving a user selection of one or more contacts in the second display area; receiving a user selection of a first parameter provided in the third display area, wherein the first parameter is indicative of a type of montage, and wherein the third display area further comprises at least a second parameter and a third parameter for selection by the user; and automatically generating said one or more montages based on the selected one or more contacts, the first parameter, and at least one of the second parameter or third parameter.

Optionally, the image is a three dimensional image of the patient's brain.

Optionally, the one or more contacts are selected from within an electrode of the one or more electrodes and/or from across the one or more electrodes.

Optionally, the type of montage is one of a bipolar montage, a referential montage or an average referential montage.

Optionally, the method of further comprises generating the one or more montages and displaying a second plurality of EEG traces corresponding to the generated one or more montages in the third display area.

Optionally, the method further comprises: receiving a user selection of a first contact of the one or more contacts; and receiving a user selection of the second parameter, wherein the second parameter defines a neural network programmatically associated with an anatomical location of the first contact and wherein additional ones of the one or more contacts are selected automatically based on the selected neural network.

Optionally, the method further comprises: receiving a user selection of the third parameter, wherein the third parameter is indicative of at least one of an anatomical region or functional region of the patient's brain, wherein a portion of the one or more contacts are associated with at least one of the anatomical region or functional region, and wherein the one or more contacts are selected automatically based on the user selection of the third parameter.

Optionally, at least one of the one or more contacts is located in white matter and at least one of the one or more contacts is positioned in gray matter. Optionally, the method further comprises receiving a user selection of a fourth parameter, wherein the fourth parameter is visually presented in the third display area and defines the at least one of the one or more contacts located in white matter as being a reference contact, and wherein the first parameter is defined as being a referential montage.

The present specification also discloses a computer-implemented method of enabling a user to generate one or more montages using a computing device, wherein the computing device comprises at least one processor configured to execute programmatic instructions and a non-transient memory adapted to store the programmatic instructions, the method comprising: generating a graphical user interface, wherein the graphical user interface comprises at least a first display area, a second display area and a third display area, wherein each of the first display area, the second display area and the third display area is separate and concurrently displayed with each other; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is co-registered with a three-dimensional coordinate system; acquiring, using the computing device, EEG data of the patient, wherein the EEG data comprises a first plurality of EEG traces; displaying, simultaneously, at least a portion of the first plurality of EEG traces in the first display area and the post-surgical data in the second display area; automatically visually associating the visual representation of the one or more electrodes and associated one or more contacts with a corresponding one of the first plurality of EEG traces; receiving a user selection of a first contact of the one or more contacts in the second display area; receiving a user selection of a first parameter displayed in the third display area, wherein the first parameter is indicative of a neural network; automatically selecting a portion of the one or more contacts, wherein the portion of the one or more contacts is programmatically associated with the neural network; receiving a user selection of a second parameter displayed in the third display area, wherein the second parameter is indicative of a type of montage; and automatically generating the one or more montages based on the automatically selected one or more contacts of interest and the selected type of montage.

Optionally, the type of montage is one of a bipolar montage, a referential montage or an average referential montage.

Optionally, the method further comprises displaying a second plurality of EEG traces corresponding to the generated one or more montages in the third display area.

The present specification also discloses a computer-implemented method of enabling a user to generate one or more montages using a computing device, wherein the computing device comprises at least one processor configured to execute programmatic instructions and a non-transient memory adapted to store the programmatic instructions, the method comprising: generating a graphical user interface, wherein the graphical user interface comprises at least a first display area, a second display area and a third display area, wherein each of the first display area, the second display area and the third display area is separate and concurrently displayed with each other; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts; acquiring, using the computing device, a first plurality of EEG traces of the patient; displaying, simultaneously, at least a portion of the first plurality of EEG traces in the first display area and the post-surgical data in the second display area; automatically visually associating the visual representation of each of the one or more electrodes and associated one or more contacts in the post-surgical data with a corresponding one of the first plurality of EEG traces; receiving a user selection of a first parameter displayed in the third display area, wherein the first parameter is indicative of at least one of an anatomical region or functional region of the patient's brain; automatically selecting a portion of the one or more contacts in the second display area, wherein the portion of the one or more contacts is associated with the selected anatomical region or functional region; receiving a user selection of a second parameter displayed in the third display area, wherein the second parameter is indicative of a type of montage; and automatically generating said one or more montages based on the automatically selected one or more contacts of interest and the selected second parameter. Optionally, the type of montage is one of a bipolar montage, a referential montage or an average referential montage. Optionally, the three-dimensional image is co-registered with a three-dimensional coordinate system. Optionally, the method further comprises displaying a second plurality of EEG traces corresponding to the generated one or more montages in the third display area.

The present specification also discloses a computer-implemented method of enabling a user to generate one or more montages using a computing device, wherein the computing device comprises at least one processor configured to execute programmatic instructions and a non-transient memory adapted to store the programmatic instructions, the method comprising: generating a graphical user interface, wherein the graphical user interface comprises at least a first display area, a second display area and a third display area, wherein each of the first display area, the second display area and the third display area is visually separate when concurrently displayed; acquiring, using the computing device, a three-dimensional image of a patient's brain comprising data indicative of a visual representation of one or more electrodes placed in the patient's brain, wherein each of the one or more electrodes has one or more contacts therewith, and wherein the three-dimensional image is co-registered with a three-dimensional coordinate system; acquiring, using the computing device, a first plurality of EEG traces of the patient; displaying, simultaneously, at least a portion of a the first plurality of EEG traces in the first display area and the post-surgical data in the second display area; automatically visually associating the visual representation of at least a portion of the one or more contacts with a corresponding one of the first plurality of EEG traces; receiving a user selection of the one or more contacts; receiving, via the third display area, a user selection of a type of montage, wherein the type of montage is a referential montage; receiving, via the third display area, a user selection of a second parameter, wherein the second parameter defines at least one of the one or more contacts as a reference contact; and automatically generating said one or more montages based on the selected one or more contacts of interest, the type of montage and the reference contact.

Optionally, at least a portion of the selected one or more contacts is positioned in gray matter. Optionally, at least a portion of the selected one or more contacts is positioned in white matter.

Optionally, the method of further comprises displaying the generated one or more montages in the second display area.

The present specification also discloses a computer-implemented method of enabling a user to generate one or more montages using a computing device, wherein the computing device comprises at least one processor configured to execute programmatic instructions and a non-transient memory adapted to store the programmatic instructions, the method comprising: generating a graphical user interface, wherein the graphical user interface comprises at least a first display area, a second display area and a third display area, wherein the first display area, the second display area and the third display area are visually separate and distinct from each other, and wherein the first and second display areas are positioned side-by-side; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is aligned with a three-dimensional coordinate system; acquiring, using the computing device, EEG data of the patient; displaying, simultaneously, the EEG data in the first display area and the post-surgical data in the second display area of the graphical user interface, wherein the EEG data is displayed as a first plurality of EEG traces; automatically visually associating the visual representation of each of the one or more electrodes and associated one or more contacts in the post-surgical data with a corresponding one of the first plurality of EEG traces; selecting one or more contacts of interest in the second display area, wherein the one or more contacts of interest are selected from within an electrode of the one or more electrodes and/or from across the one or more electrodes; selecting, by the user, a first parameter provided in the third display area, wherein the first option is indicative of a type of montage, wherein the third display area further provides second, third, fourth and fifth options for activation by the user; and automatically generating said one or more montages based on the selected one or more contacts of interest, the selected first option and activation of at least one of second, third, fourth and fifth options.

Optionally, the type of montage is one of bipolar, referential or average referential.

Optionally, the computer-implemented method further comprises generating and displaying the one or more montages in the second display area, wherein a second plurality of EEG traces corresponding to the generated one or more montages is displayed in the third display area.

Optionally, the computer-implemented method further comprises: selecting, by the user, a first contact of interest in the second display area; and activating, by the user, the second option indicative of a predefined neural network anatomically associated with a location of the first contact, wherein locations of the one or more contacts of interest are anatomically associated with the predefined neural network, and wherein the one or more contacts of interested are selected automatically as a result of activation of the second option.

Optionally, the computer-implemented method further comprises activating, by the user, the third option indicative of a predefined anatomical and/or functional region of the patient's brain, wherein the one or more contacts of interest are associated with the predefined anatomical and/or functional region, and wherein the one or more contacts of interested are selected automatically as a result of activation of the third option.

Optionally, at least one of the associated one or more contacts is located in white matter and each of the one or more contacts of interest is positioned in gray matter. Optionally, the computer-implemented method further comprises activating, by the user, the fourth option indicative of selecting said at least one of the associated one or more contacts located in white matter as a reference contact for each of the one or more contacts of interest, wherein the type of montage is referential. Optionally, the computer-implemented method further comprises activating, by the user, the fifth option, enabling a clinician to select a local white matter reference contact for each grey matter contact.

The present specification also discloses a computer-implemented method of enabling a user to generate one or more montages using a computing device, wherein the computing device comprises at least one processor configured to execute programmatic instructions and a non-transient memory adapted to store the programmatic instructions, the method comprising: generating a graphical user interface, wherein the graphical user interface comprises at least a first display area, a second display area and a third display area, wherein each of the first display area, the second display area and the third display area is separate and distinct from each other, and wherein the first and second display areas are positioned side-by-side; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is co-registered with a three-dimensional coordinate system; acquiring, using the computing device, EEG data of the patient; displaying, simultaneously, the EEG data in the first display area and the post-surgical data in the second display area of the graphical user interface, wherein the EEG data is displayed as a first plurality of EEG traces; automatically visually associating the visual representation of each of the one or more electrodes and associated one or more contacts in the post-surgical data with a corresponding one of the first plurality of EEG traces; selecting, by the user, a first contact of interest in the second display area; activating, by the user, a first option provided in the third display area, wherein the first option is indicative of a predefined neural network connected to the first contact; automatically selecting one or more contacts of interest in the second display area, wherein the one or more contacts of interest are associated with the predefined neural network and include the first contact; selecting, by the user, a second option provided in the third display area, wherein the second option is indicative of a type of montage; and automatically generating said one or more montages based on the automatically selected one or more contacts of interest and the selected second option.

Optionally, the type of montage is one of bipolar, referential or average referential.

Optionally, the generated one or more montages is displayed in the second display area and a second plurality of EEG traces corresponding to the generated one or more montages is displayed in the third display area.

The present specification also discloses a computer-implemented method of enabling a user to generate one or more montages using a computing device, wherein the computing device comprises at least one processor configured to execute programmatic instructions and a non-transient memory adapted to store the programmatic instructions, the method comprising: generating a graphical user interface, wherein the graphical user interface comprises at least a first display area, a second display area and a third display area, wherein each of the first display area, the second display area and the third display area is separate and distinct from each other, and wherein the first and second display areas are positioned side-by-side; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is co-registered with a three-dimensional coordinate system; acquiring, using the computing device, EEG data of the patient; displaying, simultaneously, the EEG data in the first display area and the post-surgical data in the second display area of the graphical user interface, wherein the EEG data is displayed as a first plurality of EEG traces; automatically visually associating the visual representation of each of the one or more electrodes and associated one or more contacts in the post-surgical data with a corresponding one of the first plurality of EEG traces; activating, by the user, a first option provided in the third display area, wherein the first option is indicative of a predefined anatomical and/or functional region of the patient's brain; automatically selecting one or more contacts of interest in the second display area, wherein the one or more contacts of interest are associated with the predefined anatomical and/or functional region; selecting, by the user, a second option provided in the third display area, wherein the second option is indicative of a type of montage; and automatically generating said one or more montages based on the automatically selected one or more contacts of interest and the selected second option.

Optionally, the type of montage is one of bipolar, referential or average referential.

Optionally, the generated one or more montages is displayed in the second display area and a second plurality of EEG traces corresponding to the generated one or more montages is displayed in the third display area.

The present specification also discloses a computer-implemented method of enabling a user to generate one or more montages using a computing device, wherein the computing device comprises at least one processor configured to execute programmatic instructions and a non-transient memory adapted to store the programmatic instructions, the method comprising:
generating a graphical user interface, wherein the graphical user interface comprises at least a first display area, a second display area and a third display area, wherein each of the first display area, the second display area and the third display area is separate and distinct from each other,
and wherein the first and second display areas are positioned side-by-side; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or
more electrodes has associated one or more contacts, and wherein the three-dimensional image is co-registered with a three-dimensional coordinate system; acquiring, using the computing device, EEG data of the patient; displaying, simultaneously, the EEG data in the first display area and the post-surgical data in the second display area of the graphical user interface,
wherein the EEG data is displayed as a first plurality of EEG traces; automatically visually associating the visual representation of each of the one or more electrodes and associated one or more contacts in the post-surgical data with a corresponding one of the first plurality of EEG traces; selecting one or more contacts of interest in the second display area, wherein the one or
more contacts of interest are selected from within an electrode of the one or more electrodes and/or from across the one or more electrodes; activating, by the user, a first option provided in the third display area, wherein the first option is indicative of selecting one of the associated one or more contacts as a reference contact for each of the selected one or more contacts of interest; selecting, by the user, a second option provided in the third display area, wherein the second option is indicative of referential montage; and automatically generating said one or
more montages based on the selected one or more contacts of interest, the activated first option and the selected second option.

Optionally, each of the selected one or more contacts of interest is positioned in gray matter.

Optionally, the generated one or more montages is displayed in the second display area and a second plurality of EEG traces corresponding to the generated one or more montages is displayed in the third display area.

Optionally, at least one of the associated one or more contacts is located in white matter.

The aforementioned and other embodiments of the present specification shall be described in greater depth in the drawings and detailed description provided below.

### BRIEF DESCRIPTION OF THE DRAWINGS

The accompanying drawings illustrate various embodiments of systems, methods, and embodiments of various other aspects of the disclosure. Any person with ordinary skill in the art will appreciate that the illustrated element boundaries (e.g. boxes, groups of boxes, or other shapes) in the figures represent one example of the boundaries. It may be that in some examples one element may be designed as multiple elements or that multiple elements may be designed as one element. In some examples, an element shown as an internal component of one element may be implemented as an external component in another and vice versa. Furthermore, elements may not be drawn to scale. Non-limiting and non-exhaustive descriptions are described with reference to the following drawings. The components in the figures are not necessarily to scale, emphasis instead being placed upon illustrating principles.
FIG. 1 is a block diagram illustrating an integrated multi-modality system, in accordance with some embodiments of the present specification;
FIG. 2 is a flowchart illustrating multiple steps in an integrated multi-modality exam workflow, focused on generating a 3D-guided montage of contacts of interest (COI), in accordance with some embodiments described in the present specification;
FIG. 3A illustrates a first unified viewing environment that displays EEG traces alongside a visual representation of the corresponding electrodes and contacts within a 3D image of a patient's brain, in accordance with some embodiments of the present specification;
FIG. 3B presents an alternate view of the first unified viewing environment shown in FIG. 3A, allowing a clinician to visualize the association between EEG and imaging data within the patient's 3D brain image and to select one or more contacts of interest, in accordance with some embodiments of the present specification;
FIG. 3C depicts an additional view of the first unified viewing environment shown in FIG. 3A, enabling the clinician to visualize the association between EEG and imaging data within the patient's 3D brain image and to select one or more contacts of interest, in accordance with some embodiments of the present specification;
FIG. 3D illustrates a second unified viewing environment that displays an anatomical atlas within a 3D brain image, in accordance with some embodiments of the present specification;
FIG. 4A depicts a third unified viewing environment designed to assist a clinician in automatically generating and displaying one or more montages in association with a visual representation of corresponding electrodes and contacts of interest within the patient's 3D brain image;
FIG. 4B displays an enlarged view of the COI window within the third unified viewing environment shown in FIG. 4A, in accordance with some embodiments of the present specification;
FIG. 4C illustrates a fifth unified viewing environment that supports a clinician in automatically generating and displaying a one or more referential montages in association with a visual representation of corresponding electrodes and contacts of interest within a patient's 3D brain image, in accordance with some embodiments of the present specification;
FIG. 4D illustrates a sixth unified viewing environment that supports a clinician in automatically generating and displaying bipolar montages within specific electrodes that are associated with an anatomical region, in accordance with some embodiments of the present specification;
FIG. 4E illustrates a seventh unified viewing environment that supports a clinician in automatically generating and displaying cross electrode bipolar montages associated with an anatomical region, in accordance with some embodiments of the present specification;
FIG. 5 depicts an approach for individually addressing each contact of interest within a first electrode and a second electrode positioned within a 3D brain image of a patient, in accordance with some embodiments of the present specification;
FIG. 6 illustrates a cortical homunculus, in accordance with some embodiments of the present specification;
FIG. 7 illustrates brain maps identifying a plurality of specific brain regions and structures, in accordance with some embodiments of the present specification;
FIG. 8 is a segmented map of the brain, in accordance with some embodiments of the present specification; and
FIG. 9 shows brain magnetic resonance imaging (MRI) and diffusion tensor tractographies (DTTs) of a patient, in accordance with some embodiments of the present specification.

### DETAILED DESCRIPTION

In embodiments, the present specification discloses systems and methods for the rapid creation of montages and EEG trace data based on contacts of interest for an interactive and dynamic exploration of a patient's electrophysiological profile. Further, EEG signals/traces are correlated with anatomical images in a unified viewing environment for supporting a clinician by optimizing an understanding of the actual source of electrical activity and its spread through the neural network of the patient's brain. In embodiments, a visual representation of each electrodes/contact(s) is automatically visually associated with EEG traces, which enables clinicians to dynamically correlate electrophysiological activity with precise neuroanatomical locations during analysis. This is enabled via several methods, including metadata matching in which electrode profiles (manufacturer-specific dimensions, contact spacing) and pre-surgical trajectory data are matched to post-surgical CT coordinates. Further, contact IDs and spatial coordinates in the 3D brain images are algorithmically linked to EEG channel labels via predefined configurations. In addition, the systems and methods of the present specification support 3D spatial alignment, in which electrodes from pre-surgical MRI plans are algorithmically adjusted to align with CT-detected contacts in the unified coordinate system. Further, the systems and methods support bi-directional GUI interaction - selecting an EEG trace highlights its associated contact in the 3D images (and vice versa). Thus, the highlighting of traces in the trace window and the highlighting of contacts in the 3D images affords the visualization in a plurality of ways, including highlighting the trace labels (montage), and/or drawing the traces in a different color or thickness, and/or changing the background color of the traces. The contacts may be highlighted by, for example, by changing the shape of the contact (to a cube for instance), and/or the color, and/or provide some glowing color around it, among various other manipulations. In embodiments, multi-trace selections trigger a collective highlighting of all related contacts across electrodes.

The present specification is directed towards multiple embodiments. The following disclosure is provided in order to enable a person having ordinary skill in the art to practice the invention. Language used in this specification should not be interpreted as a general disavowal of any one specific embodiment or used to limit the claims beyond the meaning of the terms used therein. The general principles defined herein may be applied to other embodiments and applications without departing from the spirit and scope of the invention. Also, the terminology and phraseology used is for the purpose of describing exemplary embodiments and should not be considered limiting. Thus, the present invention is to be accorded the widest scope encompassing numerous alternatives, modifications and equivalents consistent with the principles and features disclosed. For purpose of clarity, details relating to technical material that is known in the technical fields related to the invention have not been described in detail so as not to unnecessarily obscure the present invention.

In various embodiments, a computing device includes an input/output controller, at least one communications interface and system memory. The system memory includes at least one random access memory (RAM) and at least one read-only memory (ROM). These elements are in communication with a central processing unit (CPU) to enable operation of the computing device. In various embodiments, the computing device may be a conventional standalone computer or alternatively, the functions of the computing device may be distributed across multiple computer systems and architectures.

In some embodiments, execution of a plurality of sequences of programmatic instructions or code enable or cause the CPU of the computing device to perform various functions and processes. In alternate embodiments, hard-wired circuitry may be used in place of, or in combination with, software instructions for implementation of the processes of systems and methods described in this application. Thus, the systems and methods described are not limited to any specific combination of hardware and software.

The term "module" used in this disclosure may refer to computer logic utilized to provide a desired functionality, service or operation by programming or controlling a general-purpose processor. Stated differently, in some embodiments, a module, application or engine implements a plurality of instructions or programmatic code to cause a general-purpose processor to perform one or more functions. In various embodiments, a module, application or engine can be implemented in hardware, firmware, software or any combination thereof. The module, application or engine may be interchangeably used with unit, logic, logical block, component, or circuit, for example. The module, application or engine may be the minimum unit, or part thereof, which performs one or more particular functions.

The term "montage" used in this disclosure may refer to a logical arrangement of channels - each representing a comparison between two EEG amplifier inputs - that is used to convey information about electrical signals in the brain. As used in this specification, a montage can be bipolar or differential.

As used in this specification, the term "referential montage" refers to an EEG channel arrangement in which each selected contact of interest is compared against a single reference contact, so the displayed traces show activity relative to that reference rather than between adjacent contacts. More specifically, in embodiments, the specification describes referential montaging as a mode where the user selects a reference contact, and the system automatically generates traces such as A1-B2, A2-B2, and so on when B2 is the chosen reference. The reference may be dynamic, in embodiments, meaning the montage updates in real time if the user chooses a different contact as the reference.

In embodiments of the present specification, an "EEG trace" is the waveform of a montage channel created by taking the mathematical difference of two input waveforms, so a montage is essentially the channel layout that produces those traces. Further, in embodiments, montages may be created to emphasize local signal gradients, minimize noise, and help localize an abnormal event or neural source.

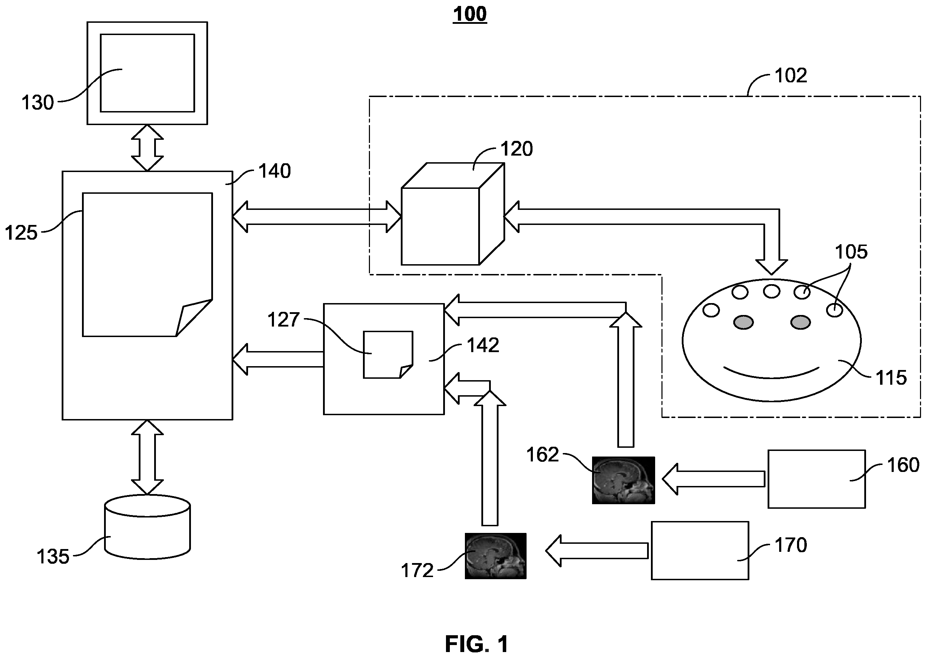

The term "average reference," as used in this disclosure, may denote a referencing method in which, for a defined region surrounding a selected contact, the electrical activity from all contacts within that region is averaged to serve as a reference for other contacts within the same space. The region may take the form of a sphere, cube, another standard geometric shape, or a custom-defined volume of interest. In some embodiments, a module (such as module 125 described with reference to FIG. 1 below) is configured to automatically select which electrodes are included in the average, based on their signal characteristics or content. If an electrode exhibits excessive activity, its signal may be excluded from the average.

As used throughout this specification, the term "virtual electrodes" is used to refer to simulated or projected electrode representations that appear in the 3D brain-image workflow before or alongside the actual implanted electrodes, allowing the clinician to plan, visualize, and map expected electrode positions and trajectories in the patient-specific brain model.

The term "patient-specific brain model" refers to a 3D brain representation created from the individual patient's imaging data and aligned to that patient's anatomy for mapping electrodes, contacts, and EEG traces, in embodiments of the present specification.

As used throughout the specification, in embodiments, "contacts of interest" or "COI" are the selected electrode contacts the user chooses for analysis, either within one electrode or across multiple electrodes, so the system can automatically generate the desired montage.

Throughout the specification, the term "view area" may also be referred to as "display area". For example, without limitation, the first view area 305a, second view area 320a, third view area 420a, fourth view area 430a, and fifth view area 431a may, respectively, be referred to as the first display area 305a, second display area 320a, third display area 420a, fourth display area 430a, and fifth display area 431a.

In embodiments of the present specification, "pre-surgical data" is the patient's processed preoperative imaging and planning data, especially the MRI-based 3D brain model plus the planned electrode placements or trajectories used before implantation. Thus, in embodiments, pre-surgical data refers to the patient-specific imaging, planning, and electrode-placement information generated before surgery and used to guide the procedure. It includes processed and co-registered pre-surgery image data-typically MRI-based 3D brain imaging-together with the surgical plan showing the intended electrode placements or trajectories, trajectory lists, and electrode profiles used to simulate where the electrodes are meant to go, in embodiments of the present specification.

In embodiments of the present specification, "post-surgical data" refers to the patient-specific information collected, generated, or co-registered after the procedure, once the electrodes have actually been implanted and the system can identify their real positions in the brain. As used in embodiments described in this specification, it includes a three-dimensional brain image showing the surgically placed electrodes and their contacts, aligned to a three-dimensional coordinate system so the implant location can be analyzed accurately.

In the description and claims of the application, each of the words "comprise", "include", "have", "contain", and forms thereof, are not necessarily limited to members in a list with which the words may be associated. Thus, they are intended to be equivalent in meaning and be open-ended in that an item or items following any one of these words is not meant to be an exhaustive listing of such item or items, or meant to be limited to only the listed item or items. It should be noted herein that any feature or component described in association with a specific embodiment may be used and implemented with any other embodiment unless clearly indicated otherwise.

The human brain contains numerous neural networks, which are groups of interconnected neurons and nerve tracts that enable communication between different brain regions and organize complex functions. The neural networks of the brain include (among others) the visual network for processing visual information; the motor network, which is responsible for planning, initiating, and coordinating voluntary movements; the somatosensory network, which involves the primary somatosensory cortex and related pathways and processes sensory information from the body, such as touch, pressure, pain, and temperature; the limbic network, which comprises structures like the hippocampus, amygdala, and parts of the cortex and is crucial for emotion, memory, and motivation; the language network which involves Broca's area (frontal lobe), Wernicke's area (temporal lobe), and the arcuate fasciculus connecting them and enables language comprehension and production; the default mode network, which is active when the brain is at rest and not focused on the outside world, but involves self-referential thinking, daydreaming, and recalling memories; the executive control network, which involves the prefrontal cortex and related areas, supporting decision-making, attention, and working memory; the sensory-motor integration network, which combines sensory input and motor output, allowing the brain to adjust movements based on sensory feedback; and the cranial nerve network, which includes the twelve cranial nerves that emerge from the brain and brainstem, each forming networks that control specific sensory and motor functions throughout the head and neck.

In embodiments, the human brain may be segmented, classified, and/or represented according to a plurality of anatomical brain regions, functional brain regions, cortical regions, subcortical regions, white matter regions, lobes, gyri, sulci, nuclei, tracts, and/or neurophysiological zones. The systems and methods of the present specification may utilize such anatomical and/or functional classifications to support automated contact selection, montage generation, electrophysiological visualization, neural network analysis, seizure localization, stimulation planning, and/or connectivity-based analysis within the unified viewing environment.

In embodiments, anatomical brain regions represented within the patient's 3D brain image include, but are not limited to, frontal lobe regions, parietal lobe regions, temporal lobe regions, occipital lobe regions, insular regions, limbic regions, cerebellar regions, diencephalic regions, brainstem regions, cortical gray matter regions, subcortical gray matter regions, and white matter regions. In some embodiments, the frontal lobe regions include the prefrontal cortex, orbitofrontal cortex, dorsolateral prefrontal cortex, ventromedial prefrontal cortex, inferior frontal gyrus, superior frontal gyrus, middle frontal gyrus, premotor cortex, supplementary motor area, primary motor cortex, frontal pole, Broca's area, and associated frontal opercular regions. In some embodiments, the parietal lobe regions include the primary somatosensory cortex, postcentral gyrus, superior parietal lobule, inferior parietal lobule, supramarginal gyrus, angular gyrus, precuneus, and parietal operculum. In some embodiments, the temporal lobe regions include the superior temporal gyrus, middle temporal gyrus, inferior temporal gyrus, temporal pole, fusiform gyrus, parahippocampal gyrus, entorhinal cortex, hippocampus, amygdala, Heschl's gyrus, Wernicke's area, and mesial temporal structures. In some embodiments, the occipital lobe regions include the primary visual cortex, visual association cortex, cuneus, lingual gyrus, lateral occipital cortex, calcarine cortex, dorsal visual stream regions, and ventral visual stream regions.

In embodiments, additional anatomical structures represented within the patient's 3D brain image include the insular cortex, claustrum, cingulate cortex, corpus callosum, fornix, internal capsule, external capsule, corona radiata, thalamus, hypothalamus, basal ganglia, caudate nucleus, putamen, globus pallidus, nucleus accumbens, substantia nigra, subthalamic nucleus, red nucleus, mammillary bodies, cerebellar hemispheres, cerebellar vermis, medulla, pons, midbrain, cranial nerve nuclei, ventricular regions, and associated white matter tracts and commissural pathways. In some embodiments, the systems and methods additionally support segmentation and identification of gyri, sulci, Brodmann areas, stereotactic coordinate-defined regions, atlas-defined structures, and patient-specific anatomical subdivisions.

In embodiments, the systems and methods of the present specification additionally support identification and selection of functionally defined brain regions and/or functional neurophysiological regions. Such functionally defined regions may correspond to regions identified according to neural activity, connectivity, sensory processing, motor processing, language processing, memory processing, executive control, emotional processing, seizure propagation, stimulation response, and/or electrophysiological characteristics. In some embodiments, functionally defined regions include motor cortex regions, premotor regions, supplementary motor regions, somatosensory cortex regions, sensory integration regions, visual processing regions, auditory processing regions, language-processing regions, speech-production regions, speech-comprehension regions, memory-processing regions, emotional-processing regions, executive control regions, attention-control regions, default mode network regions, salience network regions, limbic network regions, seizure onset zones, epileptogenic zones, seizure propagation pathways, eloquent cortex regions, and stimulation-responsive cortical and subcortical regions.

In some embodiments, the functionally defined motor regions include the primary motor cortex, supplementary motor area, premotor cortex, corticospinal pathway regions, and associated sensorimotor integration structures. In some embodiments, the functionally defined sensory regions include the primary somatosensory cortex, secondary somatosensory cortex, somatosensory association regions, pain-processing regions, proprioceptive-processing regions, and tactile-processing regions. In some embodiments, the functionally defined visual regions include the primary visual cortex, visual association cortex, dorsal visual stream regions, ventral visual stream regions, and motion-processing visual regions. In some embodiments, the functionally defined auditory regions include Heschl's gyrus, primary auditory cortex, auditory association cortex, and speech-auditory integration regions. In some embodiments, the functionally defined language regions include Broca's area, Wernicke's area, arcuate fasciculus-associated regions, expressive language regions, receptive language regions, semantic processing regions, and speech articulation regions.

It should be noted that as used herein and in the appended claims, the singular forms "a," "an," and "the" include plural references unless the context dictates otherwise. Although any systems and methods similar or equivalent to those described herein can be used in the practice or testing of embodiments of the present disclosure, the preferred, systems and methods are now described.

### Overview

FIG. 1 is a block diagram illustrating an integrated multi-modality system, in accordance with some embodiments of the present specification. In various embodiments, system 100 offers integration, visualization, manipulation, and analysis of electroencephalography (EEG) and medical imaging data. In some configurations, system 100 includes a first modality - an EEG system 102 - configured to detect, diagnose, or predict neurological events based on EEG signals. As illustrated in FIG. 1, a plurality of EEG sensors or electrodes 105 are either implanted in the patient's brain or positioned on a tissue layer such as the scalp or cortex of the patient 115. The plurality of electrodes 105 are in data communication with a multi-channel amplifier 120, which, in turn is in data communication with a first computing device 140. The first computing device 140, in turn, is in data communication with both a display unit 130 and at least one database 135.

In various embodiments, the plurality of electrodes 105 include grid, strip and/or depth electrodes where each electrode may have multiple contacts. The plurality of electrodes 105 record electrical signals (EEG signals) from the patient's brain and communicate the analog signals using a first communication link to the multi-channel amplifier 120 that amplifies the signals, converts the signals from an analog EEG data set to a digital EEG data set, and communicates the resultant digital EEG data to the first computing device 140 over a second communication link.

In some embodiments, the system 100 incorporates a second modality featuring a first imaging system 160 that generates patient-specific first image data 162 and transmits the data 162 to a second computing device 142 via a third communication link. Additionally, system 100 may include a third modality featuring a second medical imaging system 170 that generates patient-specific second image data 172 and transmits the data 172 to the same second computing device 142 over a fourth communication link.

In various embodiments, the image data generated by the first imaging system 160 and the second imaging system 170 may be derived from a range of imaging modalities, including but not limited to Magnetic Resonance Imaging (MRI), Computed Tomography (CT), Single Proton Emission Computed Tomography (SPECT), or Positron Emission Tomography (PET) imaging. In an embodiment, the second medical imaging system is an ionizing radiation-based medical imaging system. In various embodiments, each of the plurality of imaging modalities provide image data consisting of a series of images representing information in 3D space.

The second computing device 142 includes a third-party surgical planning software or applications 127 such as ROSA (Robotic Stereotactic Assistance), which are utilized to generate processed pre- and post-surgical data. In accordance with some aspects of the present specification, a clinician can import this processed data from the second computing device 142 into the first computing device 140 via a fifth communication link. In embodiments, the first, second, third, fourth and fifth communication links may be dynamic, identical or distinct, and can be implemented using either wired or wireless connections.

Each of the first computing device 140 and second computing device 142 is equipped with an input/output controller, at least one communications interface and system memory that includes both random access memory (RAM) and read-only memory (ROM). These components interface and are in communication with a central processing unit (CPU) to support the operation of each device. In various embodiments, each of the first computing device 140 and second computing device 142 may be implemented as a conventional standalone computer, or their functions can be distributed across multiple computer systems and architectures. For example, in a distributed architecture for the first computing device 140, the database 135 and processing circuitry may be located in separate units or locations, with some units dedicated to primary processing tasks and containing, at minimum, a general controller or a processing circuitry along with system memory.

### Pre-Surgical Data and Post-Surgical Data

### Pre-Surgical Data

As mentioned earlier, the second computing device 142 includes a third-party surgical planning software or application 127 that is configured to process and support co-registration of the patient-specific first image data 162 and second image data 172.

During a pre-surgical planning stage, the patient is initially imaged using the second modality to generate the first image data 162. In some embodiments, the second modality is a magnetic resonance imaging (MRI) system, and the resulting first image data 162 consists of MRI image data that provides three-dimensional spatial information.

The second computing device 142 receives MRI image data via the third communication link and processes it using third-party surgical planning software 127 (e.g. ROSA). The key processing steps include, but are not limited to, removing background noise, optimizing images by adjusting brightness/contrast levels, automated or manual brain isolation (for example by selecting, either manually or by using an automated tool, the area of the brain and removing extracorporeal data), co-registration via spatial adjustments (adjusting offsets/rotations), and/or alignment with a predefined or standard three-dimensional coordinate system. The processing steps generate processed pre-surgery image data 162 representing an MRI-derived 3D brain image such as a Cortical Surface Projection model mapping the brain's outer surface geometry.

The third-party surgical planning software 127 uses the MRI-based 3D brain model to simulate optimal electrode configurations or placement of virtual electrodes, generating a detailed surgical plan. The surgical plan provides a baseline/goal visual representation of electrode placements or trajectories including a trajectory list, where the list includes data indicative of dimensions of each electrode (length/diameter) and the spatial start/end coordinates for each electrode. Thus, the virtual electrodes are projected onto the 3D Cortical Surface Projection model to simulate/view where the electrodes should be placed, enabling visualization of the virtual electrodes.

Typically, virtual electrode types are selected from a manufacturer-specific library of electrode profiles. The selected electrodes are manually positioned on the MRI-based 3D brain model. Electrode profiles include information such as, but not limited to, electrode types (grid, depth, and other intracranial devices), physical specifications of the electrode (including dimensions, contact counts, and material composition), and optional metadata (date of first use, sterilization details, manufacturing lot). The electrode profiles enable accurate 3D model integration by providing scalable electrode rendering based on physical measurements, anatomical tagging (positioning within white/gray matter), and/or neural network associations for functional mapping. Alternatively, ROSA (Robotic Stereotactic Assistance) trajectories, which are robot-assisted electrode placement trajectories, may be imported into the third-party surgical planning software 127. The imported virtual electrodes may then be manually aligned or manipulated to match these trajectories.

Thus, the third-party surgical planning software 127 is used to generate pre-surgical data that includes a) processed and co-registered pre-surgery first image data 162, representing an MRI-based 3D brain model, and b) a surgical plan, which provides a baseline/goal visual representation of the intended electrode placements or trajectories, along with a trajectory list and electrode profiles. In various embodiments, the first image data 162 may be derived from MRI, CT, SPECT or PET scans, resulting in a 3D brain image model based on any of these imaging modalities.

### Post-Surgical Data

Following the surgical implantation or placement of electrodes in the patient's brain, a precise patient-specific 3D anatomical image is generated, visually depicting the location, position, orientation, and dimensions of the implanted electrodes. This is accomplished by using the third modality to generate the second image data 172. In some embodiments, the third modality is a CT system, and the second image data 172 consists of a series of CT images that provide three-dimensional spatial information.

The second computing device 142 receives CT image data and processes it using the third-party surgical planning software 127 (e.g. ROSA).

The key processing steps include, but are not limited to, removing background noise, optimizing images by adjusting brightness/contrast levels, automated or manual brain isolation (for example by selecting, either manually or by using an automated tool, the area of the brain and removing extracorporeal data), co-registration via spatial adjustments (adjusting offsets/rotations), and/or alignment with the same predefined or standard three-dimensional coordinate system as first image data 162. The processing steps generate processed post-surgery image data 172 representing a CT-derived 3D brain image of the patient. In various embodiments, the second image data 172 is at least one of MRI, CT, SPECT or PET based 3D brain image.

### Multi- Modality Exam Module or Engine 125

In accordance with aspects of the present specification, the first computing device 140 includes a multi-modality exam module or engine 125. Module or engine 125 executes a set of programmatic instructions that enable it to implement an integrated multi-modality exam workflow. As a result, the module 125 can record, disp0lay, analyze, and review data from the first exam modality 102, the second exam modality 160, and the third exam modality 170, presenting this information as unified, multi-modal data.

Any steps not expressly tied to a specific purpose-built hardware embodiment may be performed by a general-purpose computing device executing appropriate programmatic code. In some embodiments, the execution of programmatic instruction sequences enables or causes the CPU to perform various functions and processes. In alternate embodiments, hard-wired circuitry may be used either instead of, or alongside, software instructions to implement the processes of the systems and the methods described in this application. Therefore, the systems and methods described herein are not restricted to any particular combination of hardware and software.

### An Integrated Multi-Modality Exam Workflow

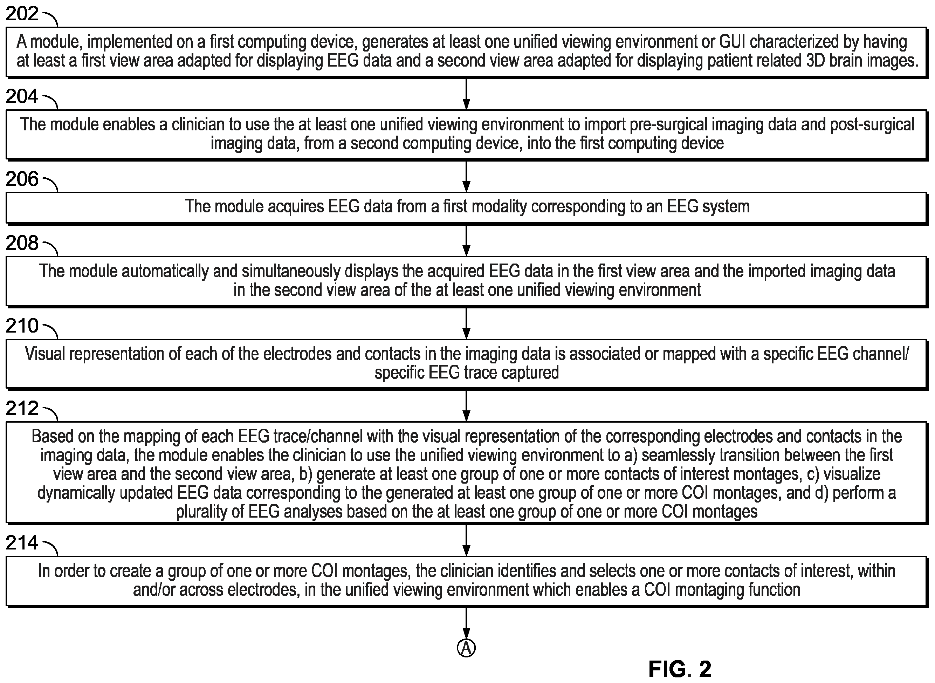

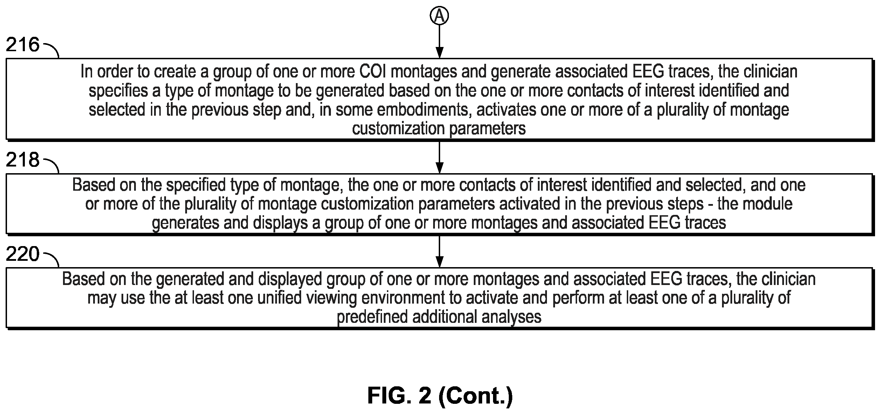

FIG. 2 is a flowchart illustrating multiple steps in an integrated multi-modality exam workflow, focused on generating a 3D-guided montage of contacts of interest (COI), in accordance with some embodiments described in the present specification. In embodiments, the module 125 is configured to implement the integrated multi-modality exam workflow 200 that harmonizes data streams for neurophysiological monitoring, structural imaging, and functional imaging.

Referring now to FIGS. 1 and 2, at step 202, module 125 generates at least one unified viewing environment or GUI (graphical user interface), characterized by at least a first view area adapted for displaying EEG data and a second view area adapted for displaying a 3D brain image of the patient. In some embodiments, the first view area is visually separate and distinct from the second view area (that is, they do not overlap). In some embodiments, the first view area and second view area are positioned side-by-side (that is, adjacent to each other). In some embodiments, the at least one unified viewing environment also includes a third view area adapted for displaying a list of electrode contacts, and optionally, metadata associated with each contact (impedance, signal quality).

At step 204, pre-configured module 125 allows the clinician to utilize the unified viewing environment to import both pre-surgical data and post-surgical data from the second computing device 142 into the first computing device 140. In some embodiments, the pre-surgical data represents a) processed and co-registered pre-surgery first image data 162, representing an MRI-based 3D brain image of the patient, and b) a surgical plan, a visual baseline or target for electrode placements or trajectories, along with a trajectory list and electrode profiles. In some embodiments, the post-surgical data consists of processed and co-registered post-surgery second image data 172, which corresponds to a CT-based 3D brain image of the patient.

The system of the present invention is advantageous is that the clinician is not required to manually pre-process or co-register the first image data 162, simulate pre-surgical electrode trajectories, or pre-process and co-register the second image data 172 within the unified viewing environment. Instead, with a single click, the clinician can import both pre- and post-surgical data directly from a third-party surgical planning software into the unified interface. This streamlined import process eliminates the need for additional manual steps, integrating with the standard clinical workflow and avoiding the time-consuming and error-prone task of manually co-registering MRI and CT-based 3D brain images or planning electrode trajectories.

Thus, the module 125 allows clinicians to import pre-surgical data and post-surgical data with no additional training required beyond the act of importing external files. In particular, the clinician does not need advanced knowledge or expertise in brain physiology mapping for this feature. As a result, "normal" epileptologists and technicians can perform the import, enhancing their clinical experience. In addition, the clinician is not required to carry out complicated co-registration or error analysis for the pre- and post-surgical data.

At step 206, the module 125 acquires EEG data collected from system 102. In embodiments, all electrode contacts are physically connected to inputs of amplifier 120 and montages are configured (channel groupings) to isolate clinically relevant EEG signals. The amplifier 120 converts analog EEG signals to digital EEG signals for analysis.

At step 208, the module 125 simultaneously renders two datasets in the unified viewing environment or GUI - the first view area for EEG display and the second view area for anatomical context. In the first view area, the real-time or recorded EEG data is shown as vertically stacked waveforms. In the second view area, the imported CT-based 3D brain image is displayed along with actual electrode placements. This highlights the spatial relationship between electrodes and brain structures. In some embodiments, the second view area may display the imported MRI-based 3D brain image along with or instead of the CT-based 3D brain image depending upon need of the clinicians.

At step 210, a visual representation of each of the electrodes and contacts in the image data (CT-based 3D brain image and/or MRI-based 3D brain image) is associated or mapped with a relevant EEG channel. This association or mapping may be achieved manually or automatically. The clinician can select contacts used to derive EEG data, select specific contacts for stimulation, enable or disable contacts to be used to derive EEG data, select contacts to be used as reference contacts, and see specific contacts anatomical locations. To enable the association, the data from each EEG trace/channel is correlated with the anatomical representation of a contact in the 3D brain images. The automatic association method employs electrode metadata (contact IDs and spatial coordinates) and matches anatomy with EEG channel labels via predefined configurations.

### Electrodes and contacts association or mapping with specific EEG traces

In some embodiments, virtual electrodes and contacts are selected from a library of manufacturer-specific electrode profiles that include dimensional specifications (length, contact spacing), material properties, and other metadata. The electrodes are automatically mapped to the planned pre-surgical imported trajectories (from the pre-surgical data) within the CT-based 3D brain image.

In some embodiments, module 125 enables alignment of virtual electrodes/contacts with their CT-visible counterparts in the 3D brain image through automatic refinement or manual calibration. The automatic refinement used algorithms to adjust virtual electrode positions to match CT-detected artifacts. The manual calibration function affords clinicians the ability to interactively adjust placements via drag-and-drop or coordinate entry.

It should be appreciated that the CT-based 3D brain image allows clinicians to visualize surgically implanted electrode contacts within a patient's brain. The module 125 uses preloaded data on electrode specifications, including type and geometric design, to map CT scan pixels to specific contacts, thereby precisely determining where each contact is located within the patient's brain. By analyzing dimensions of electrodes and the relative spacing or distribution of contacts along each electrode, the module 125 uses that information to distinguish between true electrode contacts and image artifacts or noise.

In some embodiments, the configured module 125 is used to directly compare planned electrode positions (the pre-surgical models, baseline/goal visual representations) to the actual implant locations (electrode placement, the post-surgical CT data). The comparison is used to generate and display differential measurements or deviations between where the electrodes were supposed to be and where they were actually placed, thus identifying placement errors for immediate intraoperative adjustments.

The post-surgical CT-based 3D brain image visualizations highlight the differences between planned electrode positions and actual electrode positions due to various surgical, mechanical, and anatomical factors, offering post-operative 3D validation. During surgery, surgeons may adjust electrode trajectories based on real-time observations, such as vascular avoidance or tissue resistance, robotic placement systems may introduce sub-millimeter inaccuracies in placement, and/or electrodes may flex or bend along their midsection due to pressure from surrounding brain tissue.

In some embodiments, the module 125 is configured to enable and display a real-time comparison of planned electrode trajectories versus actual implant paths within a unified 3D visualization interface. In some embodiments, the module 125 is configured to highlight, in the unified viewing environment, contacts that exceed or deviate from pre-defined positional offset (relative to at least one of the MRI or CT brain image) percentages such as, for example, above 1%, above 5%, above 10%, above 15%, above 20%, above 25%, above 30%, above 50%, above 75% or any increment therein. Accordingly, the differential image shows where, relative to the pre- or post-surgical image, the projected electrodes or implanted electrodes were actually placed thereby minimizing errors in EEG activity correlations with specific patient anatomical regions. In embodiments, the thresholds are customizable based on procedural requirements.

At step 212, the module 125 enables the clinician to use the unified viewing environment to a) seamlessly transition between the first view area displaying vertically stacked EEG traces and the second view area displaying visual representations of locations of associated electrodes and contacts in the CT-based and/or MRI-based 3D brain image of the patient, b) generate at least one group of one or more COI (contacts of interest) montages, c) visualize dynamically updated EEG data corresponding to the generated at least one group of one or more COI montages, that are linked to anatomical positions in the 3D image, and d) perform a plurality of EEG analyses (such as spectral analysis, coherence mapping, or event detection) based on the at least one group of one or more COI montages.

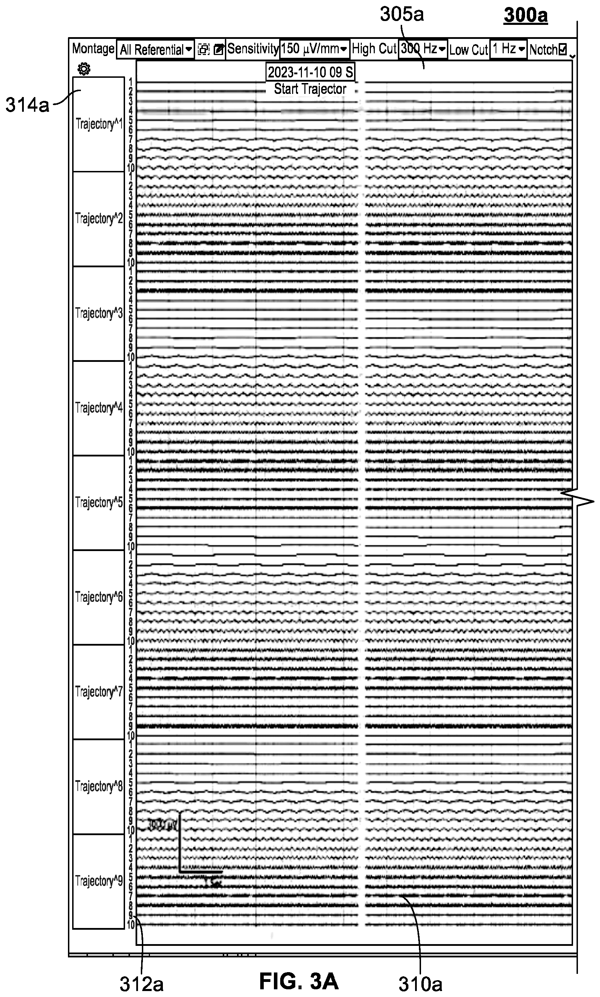

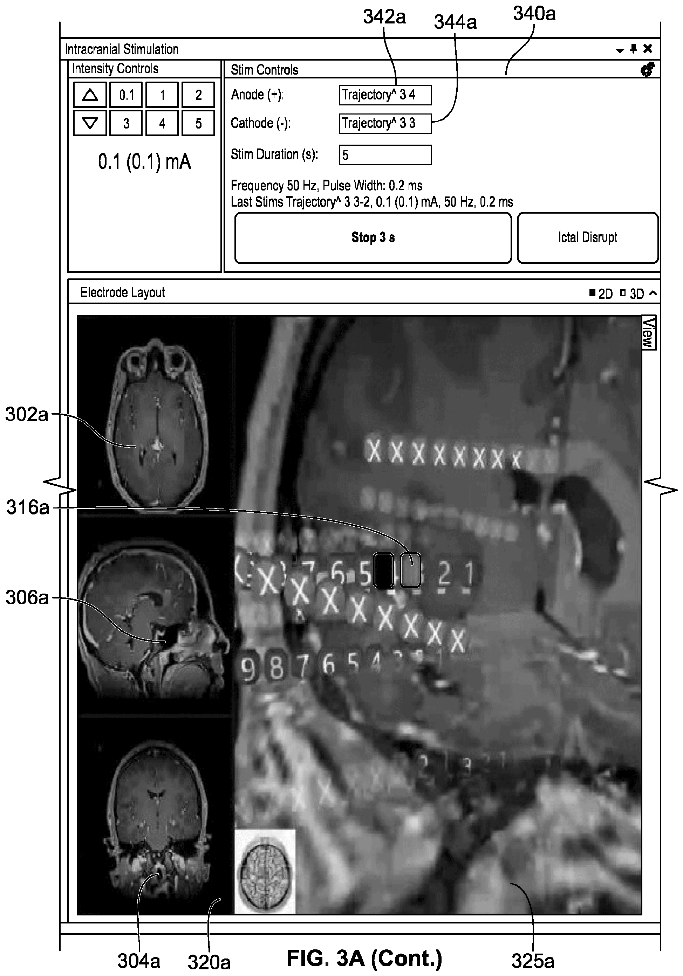

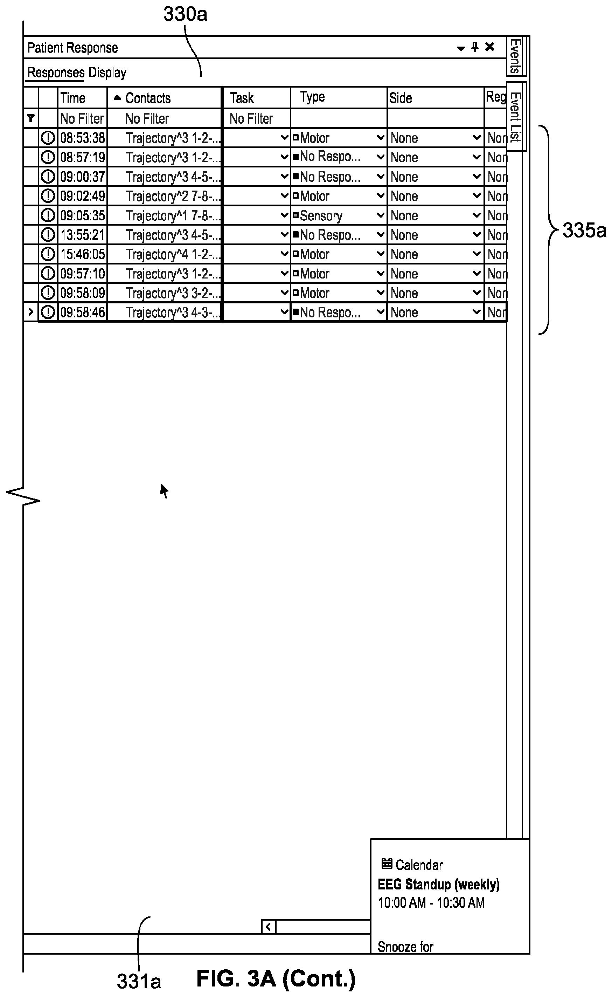

FIG. 3A illustrates a first unified viewing environment 300a that displays EEG traces alongside a visual representation of the corresponding electrodes and contacts within a 3D image of the patient's brain, in accordance with some embodiments of the present specification. In embodiments, the module 125 is configured to generate the unified viewing environment 300a that provides multimodal data visualization in a single platform, enabling a plurality of functionalities for use by a clinician. The unified viewing environment 300a comprises a first view area 305a for displaying a plurality of vertically stacked EEG traces 310a along with corresponding electrode contact identifications 312a and electrodes 314a. In embodiments, the unified viewing environment 300a comprises a second view area 320a for displaying visual representations of a plurality of electrodes and contacts in the patient brain images 325a. As described above, the actual positions of surgically implanted electrodes are extracted from the patient's post-surgical CT scan images. In some embodiments, the unified viewing environment 300a includes a third view area 330a that is used to display a list 335a of the plurality of electrode contacts and associated stimulation response characteristics of the patient. In some embodiments, the unified viewing environment 300a includes a fourth view area 340a for displaying a first drop-down list 342a that is used to select and designate an electrode contact from the plurality of electrode contacts as an anode and a second drop-down list 344a that is used to select and designate an electrode contact from the plurality of electrode contacts as a cathode. The second view area 320a also displays a first image view 302a, a second image view 304a, and a third image view 306a of the 3D brain image 325a of the patient, wherein the various numbers represent the plurality of electrode contacts.

In some embodiments, the first view area 305a is visually separate and distinct from the second view area 320a (that is, they do not overlap). In some embodiments, the first view area 305a and second view area 320a are positioned side-by-side (that is, adjacent to each other).

Referring back to FIG. 2, at step 214, clinicians generate custom contacts of interest (COI) montages by selecting contacts within and/or across electrodes in the unified viewing environment. Thus, clinicians can interactively choose one or more contacts, using a function that is provided on the unified viewing environment or interface (such as a toolbar icon), regardless of which electrodes the contacts are positioned upon, enabling a COI montaging function. In some embodiments, for example, the one or more identified contacts of interest may correspond to those indicative of potentially abnormal EEG activity.

In embodiments, one or more contacts of interest may be selected using any one of the following modes: manual COI selection mode, spatial COI selection mode, anatomical and functional COI selection mode or network/connectivity COI selection mode. In some embodiments, once selected, the contacts of interest may be highlighted in a predefined color.

### Manual COI Selection

The first mode for selecting contacts of interest (COI) is manual selection within the unified viewing environment. In this mode, the clinician can choose COIs by a) selecting EEG traces directly in the first view area (also known as a 'trace window'), b) clicking on the visual representations of contacts or virtual electrodes displayed in the 3D brain image within the second view area, or c) selecting electrode contacts from a list of electrode contacts in a third view area.

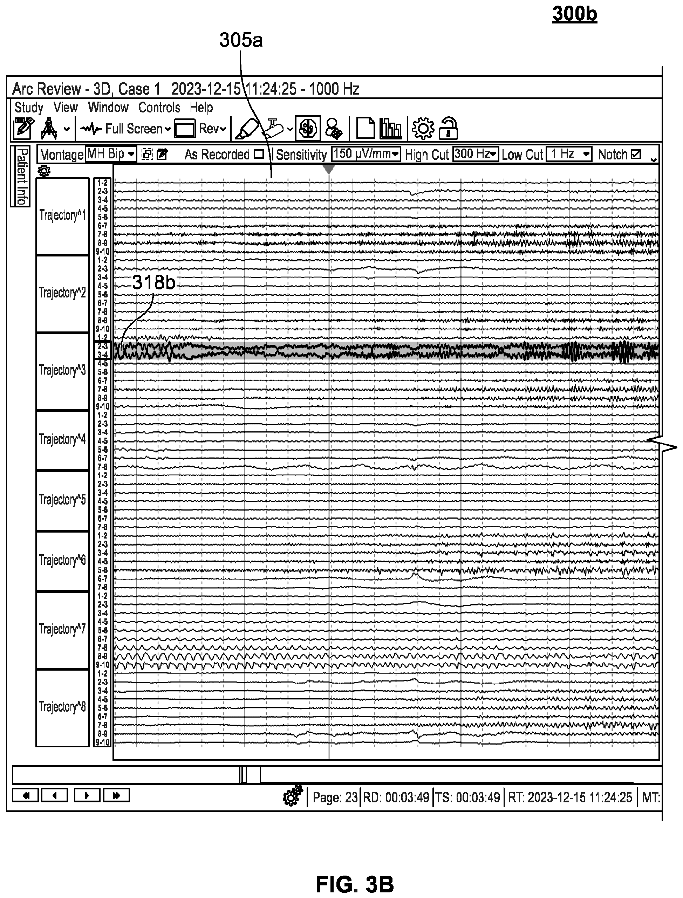

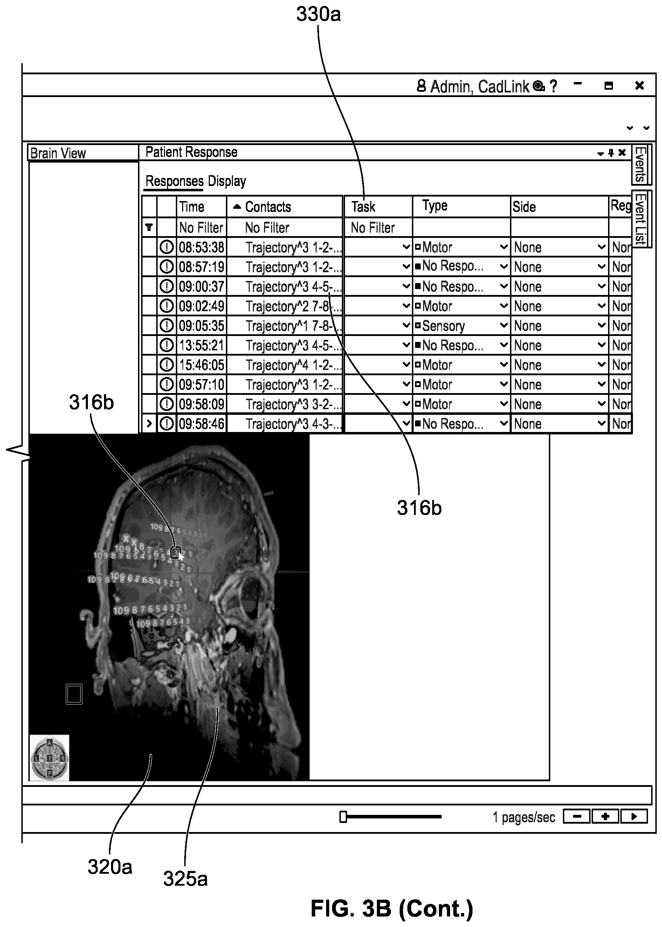

The system of the present specification links each EEG trace/channel to its corresponding electrode and contact visualization within the patient's 3D brain image, enabling clinicians to readily associate EEG activity with its anatomical origin and vice-versa. As shown in the unified viewing environment 300b of FIG. 3B, if the clinician selects (click or touch), points to, hovers over, or moves the mouse cursor over an electrode contact 316b in the 3D brain image 325a displayed in the second view area 320a, the corresponding one or more EEG trace(s) 318b, are automatically highlighted in the first view area 305a. Simultaneously, the selected associated electrode contact 316b is also automatically highlighted in the list of electrode contacts in the third view area 330a. Similarly, if the clinician selects (click or touch), hovers over, or moves a mouse cursor over an entire electrode in the 3D brain image 325a, all EEG traces associated with the contacts on that electrode are automatically highlighted in the first view area 305a. Additionally, all contacts belonging to that selected electrode are highlighted in the third view area 330a. Thus, the clinician can select an electrode contact or an entire electrode.

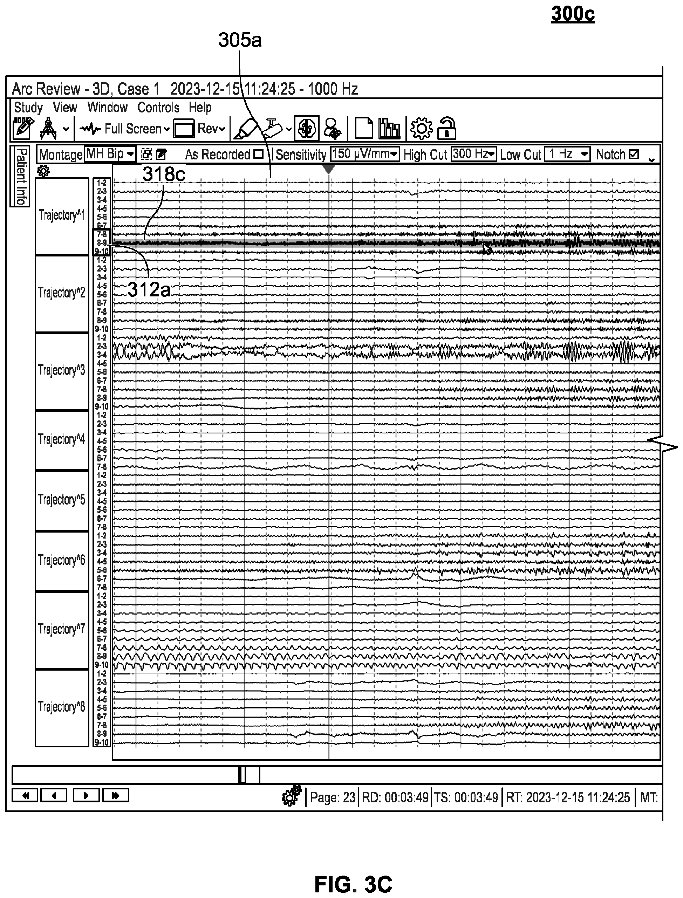

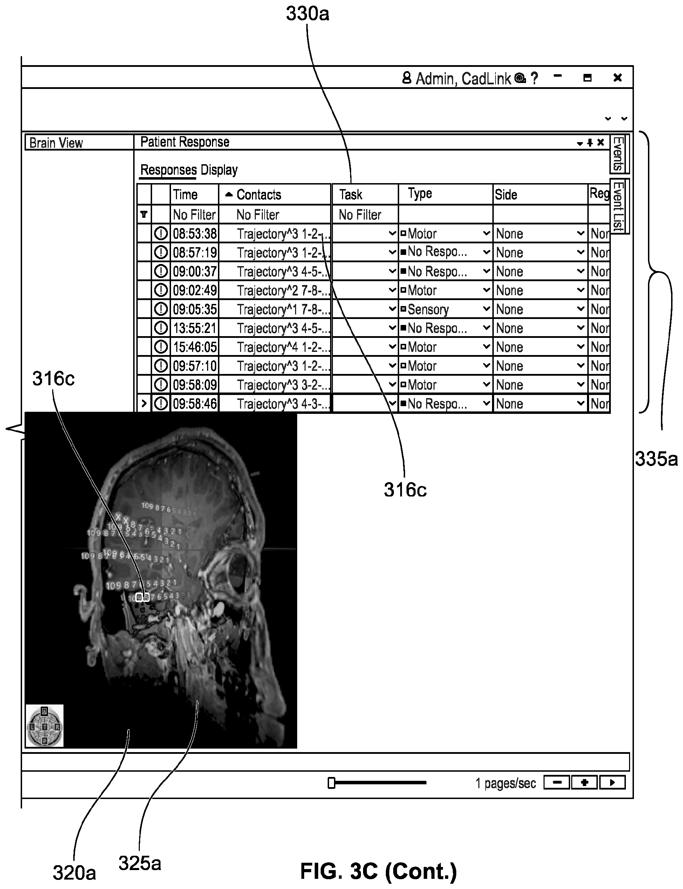

The system of the present specification also affords navigation from electrophysiological data to anatomical correlations. As shown in the unified viewing environment 300c of FIG. 3C, user interactions in the EEG display trigger corresponding highlights in the 3D image and contact lists. If the clinician selects (touch or click), hovers over, or moves the mouse cursor over an individual EEG trace 318c in the first view area 305a, the system automatically highlights the associated one or more electrode contacts 316c within the 3D brain image 325a in the second view area 320a. Concurrently, these same associated one or more electrode contacts 316c are also automatically selected or highlighted in the list within the third view area 330a. Further, if the clinician selects (touch or click), hovers over, or moves the mouse cursor over an electrode block 312a, which represents a group of traces from a single electrode, in the first view area 305a, the entire associated electrode and all of its contacts are automatically highlighted in the 3D brain image 325a in the second view area 320a. The selection is also highlighted in the list in the third view area 330a.

In addition, if the clinician selects (touch or clicks), hovers over, or moves the mouse cursor over an electrode contact within the list in the third view area 330a, the associated electrode contact is automatically highlighted in the 3D brain image 325a in the second view area 320a and the associated EEG trace is also automatically highlighted in the first view area 305a.

If the clinician selects multiple EEG traces (e.g. by using Ctrl+Click) which may or may not be contiguous, in the first view area 305a, the associated electrode contacts are automatically highlighted in the 3D brain image 325a in the second view area 320a and in the list 335a of electrode contacts in the third view 330a. In this embodiment, the clinician can use a multi-trace selection tool to select multiple EEG traces from the first view area 305a, which can be adjacent and vertically stacked atop each other (contiguous) or may have a substantial degree of separation (non-contiguous). After selection, the clinician is automatically presented with which electrode contacts in both the 3D brain image 325a and the contact list 335a correspond to each EEG trace. This allows the clinician to visualize, for any number of selected EEG traces, how they map to specific brain regions via their associated electrode contacts.

### Spatial COI Selection

The second method for selecting contacts of interest (COI) is the spatial mode, which allows clinicians to use a volume capture feature, such as lasso tool or other selection tool, to choose a group of electrode contacts directly within the patient-specific 3D brain image 325a. Close anatomical areas have traces that may be selected together however, the actual associated EEG traces may be represented in a non-contiguous manner or are presented as farther apart (due to channel ordering) in the first view area 305a. The spatial selection mode provides a method for linking anatomical regions of interest with the electrophysiological EEG traces in the first view area 305a, regardless of where the vertical traces are in relation to one another. Therefore, once a group of electrode contacts is selected in the 3D image, the unified viewing environment automatically highlights the corresponding EEG traces in the first view area 305a.

In some embodiments, clinicians can define a 3D geometrical shape or spatial region, such as a sphere, to select contacts within a specific brain volume. For instance, if a particular brain volume is suspected to be associated with seizure activity, then the volume capture function within the unified viewing environment 300c enables the clinician to isolate and examine only the contacts within that volume. In various embodiments, the defined or selected volume may be of any size or geometry.

In some embodiments, the clinician may select a target contact in the 3D brain image 325a and generate a sphere (or other volume/3D spatial region) of desired size to encompass the target brain volume around that contact. This is referred to as the bubble selection/capture volume. The action will display the associated EEG traces from contacts within that spherical volume in the first view area 305a. The clinician may then select additional contacts in the 3D brain image 325a, which results in the automatic inclusion of geographically close contacts across multiple electrodes. This selection may be further customized to reposition, add, or remove contacts from the group as needed.

In some embodiments, the system of the present specification provides two approaches for defining a 3D spatial region around the target contact for electrophysiological analysis. In a first approach, the sphere creation is parameter-drive. The sphere is generated by manually inputting (in a related dialog box, for example) at least a first parameter specifying of a maximal permissible distance to a contact neighboring the selected target contact and a second parameter specifying a radius of the sphere. In embodiments, each of the first parameter and the second parameter ranges from 5 mm to 50 mm. Module 125 is configured to automatically generate a sphere based on these parameters. In a second approach, the clinician is supported with the ability to manually create or draw and adjust (expand/collapse) the sphere (using the lasso tool), around the selected target contact, of a desired size. In embodiments, the system provides real-time visual feedback.

Thus, module 125 enables clinicians to define custom 3D spatial regions or volumes (e.g. spheres, cubes) of interest around a selected target electrode contact for focused EEG analysis. In an embodiment, a target contact may be selected by selecting an EEG trace in the first view area 305a or by selecting an electrode contact in the 3D brain image 325a. The sphere tool may be used to define the custom region (volume or 3D spatial region) around the selected target electrode contact. In some embodiments, all contacts encompassed within the 3D spatial region are automatically selected (irrespective of which electrodes these contacts belong to). As a result, the associated EEG traces in the first view area 305a are also automatically highlighted or shown. Thereafter, the clinician may click on specific contacts to deselect/reselect as desired (via sphere selection feature, for example), updating the final selection of contacts within the 3D spatial region for subsequent montaging and analyses.

In embodiments, module 125 supports simultaneous definition of multiple 3D spatial regions (e.g. spheres, cubes) around distinct target electrode contacts for comparative or additive analysis. A clinician is able to define, in embodiments, overlapping or non-overlapping 3D regions around multiple targets to isolate EEG activity from anatomically distinct areas. In other embodiments, a clinician is able to select a new target contact while deselecting at least one prior one, resulting in the automatic updating of the spatial region, highlighting EEG traces from the newly encompassed contacts.

### Anatomical and Functional COI Selection

In some embodiments, the module 125 is configured to enable the categorization and grouping of electrode contacts by anatomical region (brain anatomy) or brain function (functional networks), rather than grouping contacts based on their physical arrangement. A contact (or small metallic surface) is a single point of recording and is associated with a single channel. Contacts are mechanically/physically connected with one another vertically along a single trajectory to form a linear electrode or in a grid fashion to form a grid electrode. Conventionally, all mechanically associated contacts are grouped together in a single hardware group.

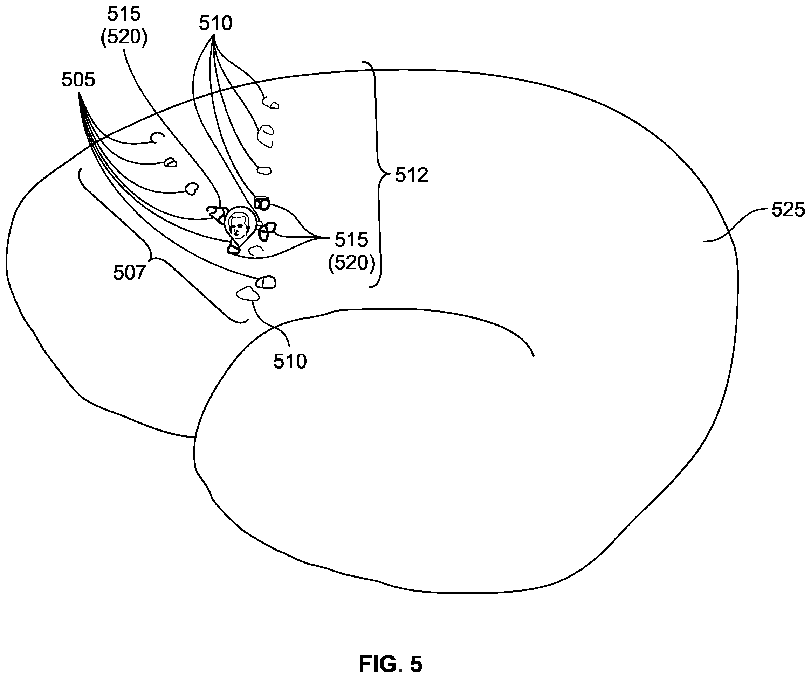

FIG. 5 depicts an approach for individually addressing each contact of interest within a first electrode and a second electrode positioned within a 3D brain image of a patient, in accordance with some embodiments of the present specification. As illustrated in FIG. 5, the system enables clinicians to treat individual contacts as independent units. A first plurality of contacts (contact array) 505 are grouped together as a first single electrode 507 and a second plurality of contacts (contact array) 510 are separately grouped together as a second single electrode 512. The first electrode 507 and second electrode 512 are visualized within a 3D brain image 525. In embodiments, module 125 is configured to allow clinicians to treat each of the contacts of the respective plurality of contacts individually and independently of other contacts that are positioned on the same electrode. Therefore, in some embodiments, the clinician can group together contacts from one electrode (for example, one or more of the first plurality of contacts 505 of first electrode 507) and contacts from another electrode (for example, one or more of the second plurality of contacts 510 of second electrode 512), creating hybrid contact groupings, such as third plurality of contacts 515 which are used to form new functional group 520. In an embodiment, the selection may be achieved by using a dialog box to input the names of the contacts or by touching/selecting the visual representations of the contacts on the screen.

The new group 520 (comprising the third plurality of contacts 515) may be based upon an anatomical region, the position of the contacts in gray matter or white matter, or the location of the contacts in a region of the brain associated with a specific function. In various embodiments, once grouped, the channels associated with those contacts (that is, the third plurality of contacts 515) may then be shown separate from the other channels, either in a new graphical user interface (GUI) view area/window or in a defined section. Referring back to FIG. 3A, in an embodiment, the unified viewing interface may include a separate view area 331a for displaying the channels associated with the third plurality of contacts 515. In an alternate embodiment, there may be a separate portion within the existing view area 305a that is dedicated to displaying the channels associated with the third plurality of contacts 515. This ability to treat contacts independent of the hardware group or electrode with which the contact would otherwise be associated is uniquely advantageous to the clinician, supporting cross-electrode network analysis.

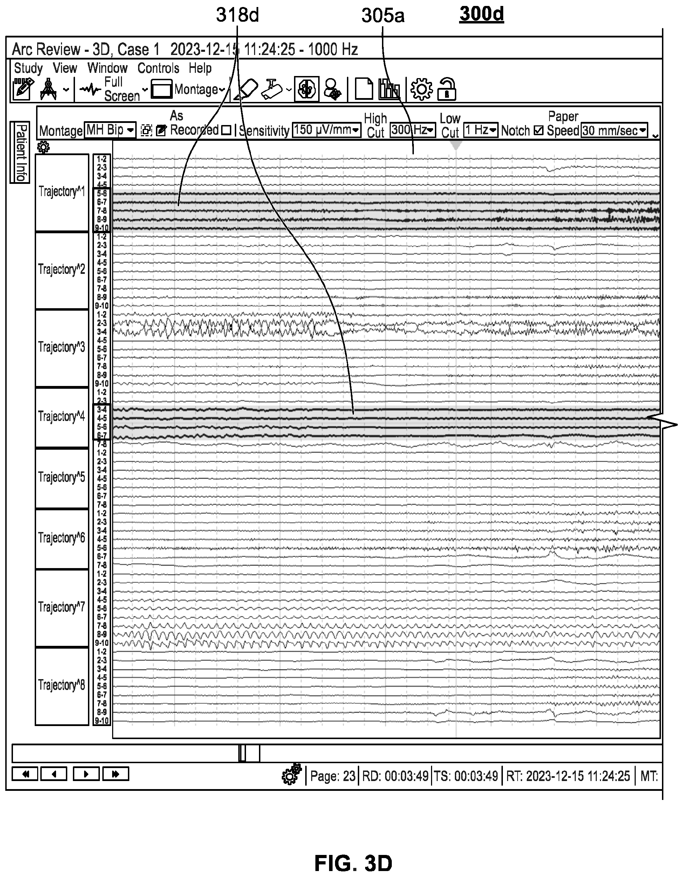

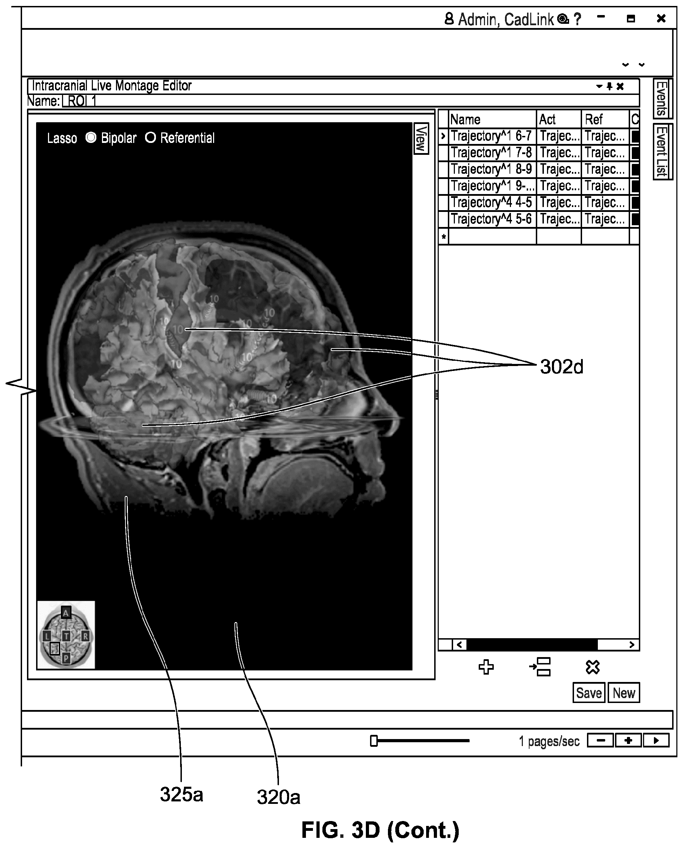

In some embodiments, the systems and methods of the present specification support an anatomical atlas-driven EEG analysis. In some embodiments, the module 125 is configured to integrate a predefined anatomical atlas into the 3D brain image, enabling clinicians to select brain regions by anatomy or function rather than electrode hardware groupings. FIG. 3D illustrates a second unified viewing environment 300d that displays an anatomical atlas within a 3D brain model 325a, in accordance with some embodiments of the present specification. The anatomical atlas is used to illustrate anatomical regions 302d, in a second view area 320a, that may be differentiated from one another and overlaid upon the 3D brain image 325a. In one embodiment, the regions 302d may be differentiated based on color-coding, using a predefined color-scale. In response to a clinician selecting at least one of the anatomical regions 302d, all electrode contacts 318d within the selected anatomical region 302d across electrodes are automatically highlighted alongside their EEG traces in the first view area 305a. In some embodiments, individual contacts can be included/excluded to refine and tailor the montage to further examine and define an anatomical and/or functional region.

In other embodiments, module 125 enables clinicians to select predefined anatomical/functional regions from a list integrated into the 3D brain image interface. The selection of a predefined anatomical and/or functional region, from the list, automatically results in grouping of all electrode contacts associated with the anatomical and/or functional region for analysis.

The clinician can choose to automatically exclude contacts located in white matter or those identified as having low or poor signal quality. Contacts of poor quality are unreliable for representing brain activity and may be excluded for several reasons: the contact may have high impedance and thus poor connection to brain tissue, may not be positioned in gray matter (which is essential for recording neuronal signaling), may be located outside the brain, or may display electrical activity that is statistically inconsistent with neighboring electrodes, such as exhibiting a low signal-to-noise ratio. In some other embodiments, clinicians have access to multiple predefined groupings of electrodes and contacts that are organized according to their anatomical and functional associations within the brain. These groupings are sourced from open-source databases, which are normalized from comprehensive libraries and made available through the anatomical atlas or as lists of predefined anatomical and functional regions within the 3D brain model.

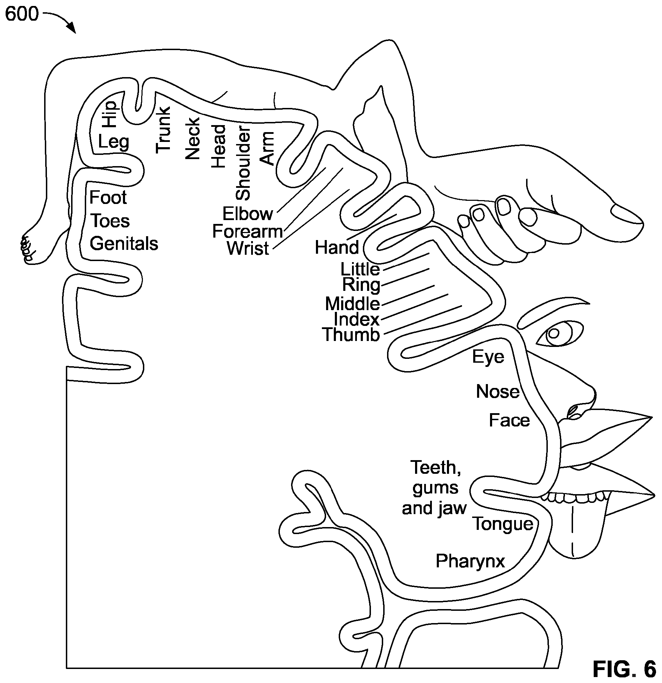

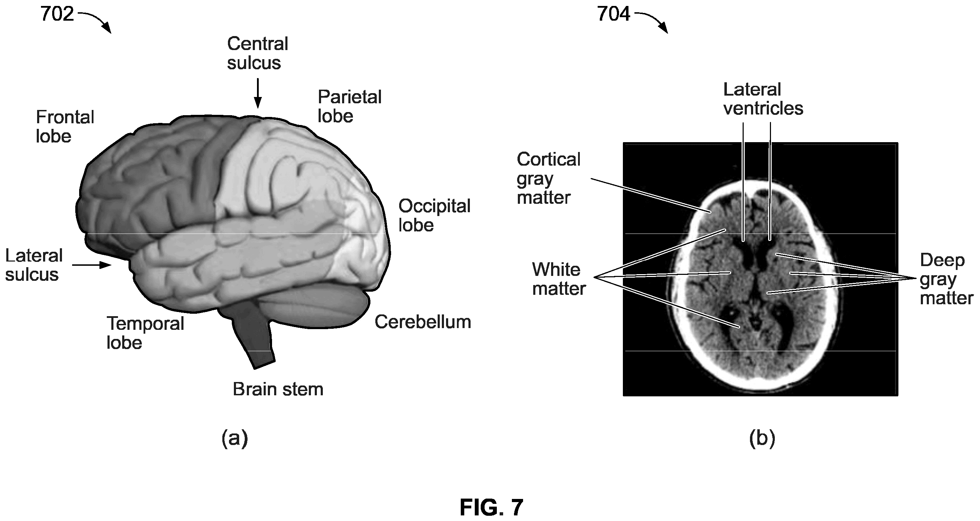

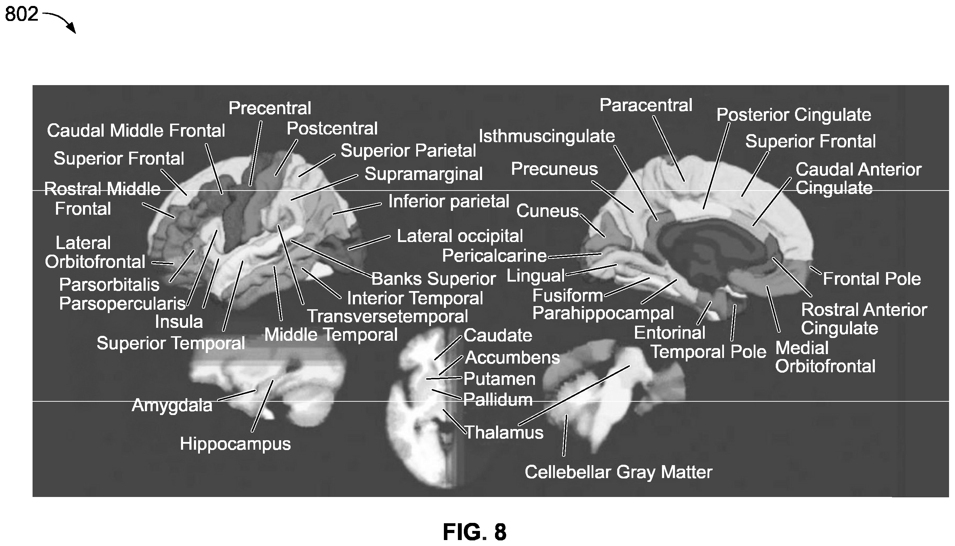

As an example of open-source data used to define anatomical and functional regions, FIG. 6 illustrates a cortical homunculus 600. This homunculus 600 is a neurologically derived map that visually represents the areas of the human brain responsible for processing motor and sensory functions for different body parts. Somatosensory nerve fibers from across the body terminate in specific regions of the parietal lobe within the cerebral cortex, creating a detailed representational map that links distinct brain areas to corresponding bodily functions. Another example of open-source data defining anatomical and/or functional brain regions is shown in FIG. 7. FIG. 7 shows two complementary maps - a first brain map 702 identifying a plurality of specific brain regions (cortical and subcortical) and a second brain map 704 identifying different brain structures (e.g. thalamus, hippocampus). A third example of open-source data for predefined anatomical and functional regions is shown in FIG. 8, which presents a segmented brain map 802 delineating 68 cortical and 16 subcortical regions of interest (ROIs). These maps, sourced from normalized neuroimaging databases, are integrated into the anatomical atlas of module 125 to guide electrode contact grouping based on functional or structural criteria.

In embodiments, by linking each EEG trace or channel to its corresponding electrode and contact within the patient's 3D brain image, the system allows for assessing contact quality and data validity/reliability. Module 125 can automatically detect and highlight potentially noisy or poor-quality channels (and associated electrodes and contacts) based their precise anatomical locations in the brain. Alternatively, or additionally, the module 125 is configured to allow a clinician to manually review, identify, and select noisy or problematic channels. The system can either automatically remove the noisy or problematic (bad) channels from analysis or enable the clinician to manually exclude them.

In some embodiments, module 125 employs machine-learning algorithms to detect poor quality EEG channels by analyzing a plurality of EEG signal features or characteristics. The plurality of signal features includes statistical metrics (variance, deviation, amplitude), correlation measures (spatial-temporal correlation coefficients), and nonlinear properties (Hurst exponent, kurtosis). Since the number of bad channels is usually much lower than the number of good channels, in some embodiments, the module 125 implements an ensemble bagging machine learning/classifier model.

Module 125 implements a three-step method to identify low-quality EEG channels. In a first step, the classifier model is trained. Historical EEG datasets where bad channels were labeled are used to train a bagging model on features, as described above. At a second step, the same signal metrics are derived from new EEG datasets and, at a third step, the metrics are provided as input to the trained classification model, to predict bad channels.

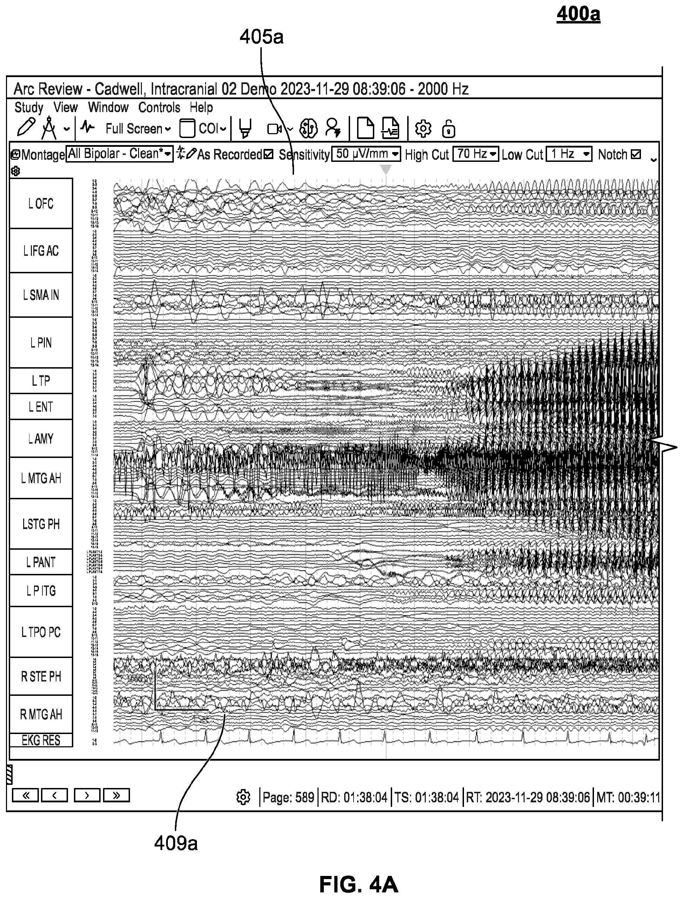

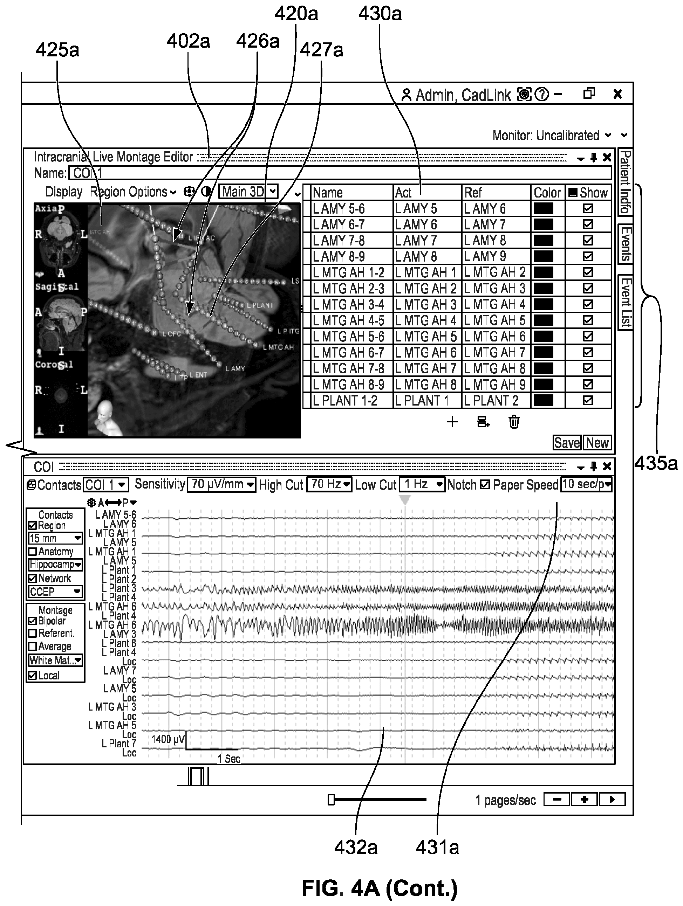

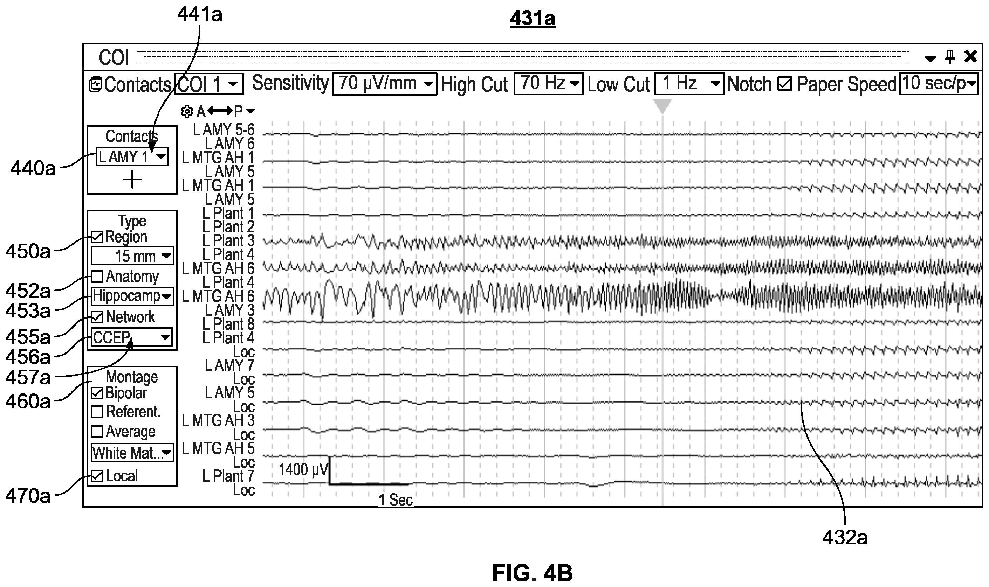

FIG. 4A depicts a third unified viewing environment 400a designed to assist a clinician in automatically generating and displaying one or more montages in association with a visual representation of corresponding electrodes and contacts of interest within the patient's 3D brain image. FIG. 4B displays an enlarged view of the COI window within the third unified viewing environment shown in FIG. 4A, in accordance with some embodiments of the present specification. The unified viewing environment, as shown in FIGS. 4A and 4B, features multiple view areas (display areas) that support comprehensive EEG analysis. In embodiments, the first view area 405a presents EEG traces 409a that correspond to the virtual representations of selected electrodes and contacts 427a. The second view area 402a is adapted to display a live montage editor and incorporates a third view area 420a, which in turn, is adapted to display the patient's 3D brain image 425a along with visual representations of electrodes, contacts, and related montaging information. A fourth view 430a is adapted to display a list of one or more montages 435a, while a fifth view area 431a is configured to display at least one COI window. The at least one COI window is configured to display EEG traces 432a corresponding to the list of one or more montages 435a, thus enabling clinicians to correlate EEG data with anatomical and montage-based groupings.

By way of non-limiting example, the 3D brain image 425a displays cross-electrode montaging configurations, enabling clinicians to visualize anatomically and functionally grouped contacts of interest (COI) based on COI selection by the clinician. The fifth view area 431a is used to dynamically display EEG traces 432a from currently selected COIs 427a, bypassing conventional montage presets. In an embodiment, clinicians can save active, configured COI groups with user-defined names, which can be recalled when needed. In embodiments, 3D image positions and EEG trace associations are included in the saved configurations. Stated differently, the unified viewing environment allows the clinician to save a configuration of selected contacts with a clinician definable name for later recall. In embodiments, the fifth view area 431a enables clinicians to arrange EEG traces from COIs using a plurality of spatial schemes, independent of physical electrode groupings, since the COIs 427a are not necessarily grouped per EEG electrode. For example, as shown, fifth view area 431a includes a listing based on anatomical axes (anterior-posterior, medial-lateral), based on an order of electrode (implantation order), or based on functional networks. In some embodiments, a clinician may use a drop-down menu to select a preferred EEG trace ordering scheme. The fifth area 431a may be configured to support simultaneous display of multiple COI windows, whereby each is linked to a distinct montage group.

In some embodiments, the first view area 405a, second view area 402a, and fifth view area 431a are visually separate and distinct from the second view area 320a (that is, they do not overlap). In some embodiments, the first view area 405a and second view area 402a are positioned side-by-side (that is, adjacent to each other). In some embodiments, the third view area 420a is visually separate and distinct from the fourth view area 430a (that is, they do not overlap). In some embodiments, the third view area 420a and fourth view area 430a are positioned side-by-side (that is, adjacent to each other) within the second view area.

In some embodiments, fifth view area 431a provides a plurality of parameters within the COI window to customize montage creation (also referred to as 'a plurality of montage customization parameters') for EEG analyses. The different check boxes/parameters (450a, 452a, 455a), which will be described in greater detail below, represent different ways of automatically selecting contacts of interest to be used in generating the montage used to display the EEG traces. The region check box (450a) is used to include all contacts located within a sphere of a predefined distance from the currently selected contact (center contact). The anatomy checkbox (452a) is used to select contacts within the same anatomical structure (for example, hippocampus). The network checkbox (455a) is used to select contacts that are part of the same neural network as the selected contact (center contact). These selections are independent of each other. A user can select to only include contacts within the same region (15mm) of the selected contact and/or contacts that are in the same anatomical location as the selected contact, and/or contacts that are part of the same neural network.

A first parameter 450a corresponds to a type of region with a dropdown menu of one or more values (in mm). The region check box (450a) is used to include all contacts located within a sphere of a predefined distance from the currently selected contact (center contact). Thus, the value defines the radius of the sphere in which contacts are to be included. In an embodiment, the predefined distance ranges from 5 mm to 50 mm, and is in an embodiment, 15mm.

A second parameter 452a, when activated (such as, for example, by clicking an associated check-box), allows the clinician to select an anatomical and/or functional region, from a dropdown list 453a of predefined anatomical and/or functional regions, within the 3D brain image 425a. This results in the selection of all electrode contacts in the 3D brain image 425a that are within the selected anatomical and/or functional region for analysis.

### Network/Connectivity COI selection

In some embodiments, module 125 enables clinicians to select groups of contacts based on predefined neural network or connectivity data. Referring back to FIGS. 4A and 4B, a third parameter 455a, when activated (such as, for example, by clicking an associated check-box), allows the clinician to select a neural network or neural connectivity data 457a, from a dropdown list 456a of one or more predefined neural networks or neural connectivity data 457a, within the 3D brain image 425.

In some embodiments, the clinician may first identify a primary contact 441a of interest from at least one of: a) a list of contacts (440a); b) directly by clicking the contact's representation in the 3D brain image 425a, or c) selecting the corresponding EEG trace in the first view area 405a. Thereafter, the clinician may activate a specific parameter, namely, the third parameter 455a (neural network selection parameter) and select the neural network or neural connectivity data 457a, from the dropdown list 456a. Examples include but are not limited to CCEPs (cortico-cortical evoked potentials), f-tract (functional brain tractography), and MRI Tractography. Upon selection of the neural network or neural connectivity data 457a, the system is configured to automatically highlight all contacts within the 3D brain image 425a that are considered part of the chosen neural network and are connected to the initially selected contact 441a.

In another embodiment, the clinician may first activate the third parameter 455a, select the neural network or neural connectivity data 457a from the list, and thereafter select the primary contact 441a. In using this sequence, the system is also configured to automatically highlight all contacts within the 3D brain image 425a that are considered part of the chosen neural network and are connected to the initially selected contact 441a. This enables a targeted analysis of EEG activity within specific, functionally or structurally defined neural circuits connected to a point of interest.

Montaging based on contacts associated with a specific neural network or connectivity allows clinicians to group electrodes and contacts according to their involvement in the same functional brain area or network, rather than being limited to traditional bipolar or referential configurations that rely on physical proximity or a shared reference electrode.

In some embodiments, the module 125 is configured to programmatically associate a specific neural network of the brain with an anatomical location of an electrode contact by algorithmically linking spatially localized electrode contact coordinates to one or more predefined functional and/or structural brain neural networks stored within the database 135 comprising, for example, atlases, connectivity maps, tractography datasets, electrophysiological response datasets, and/or machine-readable anatomical models. More specifically, once an electrode contact is localized within the 3D brain image 425a, the module 125 is configured to determine the anatomical structure, cortical region, white matter tract, and/or functional zone corresponding to that electrode contact location. The module 125 thereafter programmatically queries one or more neural connectivity datasets, from the database 135, to identify neural networks with historic, structural, functional, and/or electrophysiological association with that anatomical location. Thus, in some embodiments, the selected neural network is not manually assigned by the clinician for each electrode contact individually, but instead is automatically inferred, retrieved, and/or determined based on the anatomical position of the selected electrode contact within the patient's brain.

In some embodiments, the programmatic association is based on structural connectivity information derived from, for example, MRI tractography, diffusion tensor imaging (DTI), diffusion spectrum imaging (DSI), cortico-cortical evoked potential (CCEP) testing, functional MRI datasets, electrophysiological propagation models, atlas-based neural connectivity maps, and/or previously stored patient-specific connectivity information. Thus, when the clinician selects an electrode contact within, for example, the first view area 305a or the second view area 320a, the module 125 determines which predefined neural network is associated with the anatomical location of that electrode contact and automatically identifies additional contacts that participate in the same network. The additional contacts may be distributed across multiple electrodes, multiple lobes, contralateral hemispheres, and/or anatomically distant regions, while still being functionally or structurally connected within the same neural network or neural circuit.

By way of a first example, an electrode contact may be localized within the mesial temporal lobe, such as within the left amygdala and/or hippocampal formation. Upon localization of the electrode contact within this anatomical region, the module 125 may programmatically associate the electrode contact with a limbic neural network that includes, for example, the hippocampus, parahippocampal gyrus, entorhinal cortex, amygdala, cingulate cortex, fornix pathways, and related temporal-limbic connectivity structures. In some embodiments, the associated neural network may additionally include contacts that are identified as having electrophysiological connection using CCEP testing and/or structurally connected contacts identified using tractography data. Consequently, when the clinician activates the third parameter 455a, the module 125 (as it is configured to do so), automatically selects and/or highlights additional contacts positioned along the limbic circuitry, even where such contacts are anatomically distant from the originally selected contact. Conversely, if the same contact were instead localized within a non-limbic anatomical region, such as the dorsolateral prefrontal cortex, supplementary motor area, or occipital cortex, the associated neural network would differ substantially and would no longer correspond to the limbic network. Rather, the module 125 associates the contact with one or more different neural networks corresponding to the newly identified anatomical location.

By way of a second example, an electrode contact may be localized within a language-processing region, such as Broca's area within the inferior frontal gyrus of the dominant hemisphere. In response, the module 125 may programmatically associate the electrode contact with a language network that includes Broca's area, Wernicke's area, arcuate fasciculus pathways, superior temporal regions, premotor speech regions, and associated cortical and subcortical language-processing structures. Accordingly, activation of the third parameter 455a causes automatic identification and/or highlighting of additional contacts positioned along the language network, including contacts located within distant temporal and parietal regions that are functionally connected to the selected frontal contact. However, if the selected contact were instead positioned within the primary visual cortex of the occipital lobe, the module 125 would programmatically associate the electrode contact with a visual processing neural network involving occipital visual cortices, visual association regions, thalamic visual relay pathways, and related dorsal and ventral visual streams, thereby resulting in automatic selection of a substantially different group of contacts.

In some embodiments, the module 125 incorporates at least one artificial neural network and/or machine learning model trained using anatomical imaging data, electrophysiological recordings, tractography data, connectivity datasets, functional imaging datasets, stimulation-response datasets, and/or previously labeled neural network mappings to enable automated association between anatomical locations of electrode contacts and corresponding neural network structures within the brain. The artificial neural network may be trained to identify probabilistic relationships between localized contact coordinates and one or more functional and/or structural neural circuits, thereby enabling automated prediction, classification, and/or selection of contacts associated with related neural networks based on the detected anatomical position of a selected contact.

### Montage Parameter Selection

Referring back to FIG. 2, at step 216, the clinician specifies a type of montage (bipolar, referential or averaged/local white matter reference) to be generated based on the one or more contacts of interest identified and selected in step 214, and in some embodiments, activates one or more of a plurality of montage customization parameters.

It should be appreciated that the generation of EEG traces is dependent on a type of montage (and reference electrode selection in case of referential montage, for example). In some embodiments, in case of referential montaging, for example, the clinician can opt to have referential EEG traces displayed, updated dynamically, with one or multiple reference electrode selections.

Thus, referring back to FIG. 4B, a fourth parameter 460a (montage selection parameter) enables the clinician to select (such as, for example, by clicking an associated check-box) one of the following types of montages to be automatically generated by the module 125: bipolar, referential or averaged/local white matter reference.

A fifth parameter 470a (white matter reference parameter), when activated (such as, for example, by clicking an associated check-box), allows the clinician to reference each gray matter contact to the nearest adjacent contact in white matter tissue (select a local white matter reference contact for each grey matter contact) providing a potentially more accurate representation of the electrical activity generated at that specific electrode position.

### Bipolar Montages

Module 125 is configured to support the clinician in generating bipolar montages from contacts of interest spanning multiple electrodes, wherein customization is governed by at least one spatial or functional parameter, such as first parameter 450a, second parameter 452a, third parameter 455a, fourth parameter 460a and fifth parameter 470a. In some embodiments, spatial proximity is limited by a maximum inter-contact distance, for valid bipolar pairs. In some embodiments, the maximum inter-contact distance is user-defined and ranges from 5 mm to 50 mm, but may be upwards of 50 mm, if the user requires a larger distance. In some embodiments, the distance is less than 10 mm.

Referring back to FIGS. 4A and 4B, a clinical workflow in which a user generates bipolar montages provided by way of example. In an embodiment, unified viewing environment 400a shows that the clinician has selected the contact 441a (which, in this example, is associated with the left amygdala and referred to as 'L AMY 1' in the figures) from the list of contacts 440a. Further, the clinician has activated the following exemplary combination of parameters: the first parameter 450a specifying a type and/or size of region, the third parameter 455a specifying the neural network or neural connectivity data 457a (which in this example, is the neural network information gathered from CCEP testing) with reference to the contact 441a ('L AMY 1'), the fourth parameter 460a specifying the type of montage as bipolar, and the fifth parameter 470a specifying the type of reference electrode to be used as local white matter reference contact. It should be noted that the CCEP (Cortico Cortical Evoked Potentials) testing is a method by which a pair of contacts are used to stimulate the brain and measure the responses in the other contacts correlated to this stimulation to recognize neural network/connectivity. This information is then used to select contacts that are connected to each other (parameter 455a). The module 125 is configured to, based on these selections, automatically a) select and highlight all contacts (within and/or across electrodes), in the 3D brain image 425a that fulfills the selected criteria and b) generate a montage. The module is also configured to display the corresponding EEG traces based on the selection of the type of montage (Bipolar, Referential, Average) and the reference contact type to be used as a system reference, such as Bipolar (nearest sequential contact), Referential (a single user selectable reference contact), Average (calculated average activity of a number of user defined contacts), or a Local White Matter contact (nearest contact localized in white matter). In embodiments, the clinician may deselect some of the automatically selected contacts and/or select additional contacts from the 3D brain image 425a. Final bipolar montages and the corresponding traces are generated and displayed in the COI window in the fifth view area 431a, showing activity/EEG traces connected to the amygdala network together with contacts within the specified region (15mm).

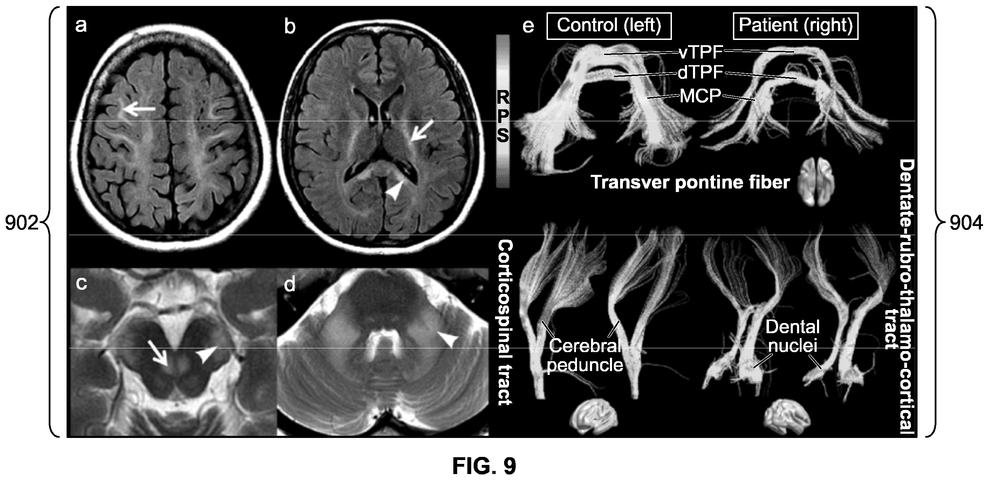

Seizure activity can spread along specific neural pathways or functional areas, as well as to nearby anatomical regions that may belong to different neural networks. In some embodiments, the unified viewing environment 400a can help clinicians identify and analyze these pathways. In embodiments, the systems and methods of the present specification support the clinician a) to stimulate a particular contact and observe responses on other contacts, b) use fMRI to map brain tracts, c) monitor the progression of electrical activity across contacts using traditional montages (bipolar, referential), and d) refine the montage to include the earliest affected contacts within a targeted neural pathway group. This allows the clinician, in embodiments, to trace the origin of a seizure, review how the seizure propagates through the brain, and identify contacts that reflect both the onset and spread of seizure activity. As a non-limiting example, FIG. 9 illustrates how brain MRI 902 and diffusion tensor tractographies (DTT) 904 can be used to map these pathways in a patient.

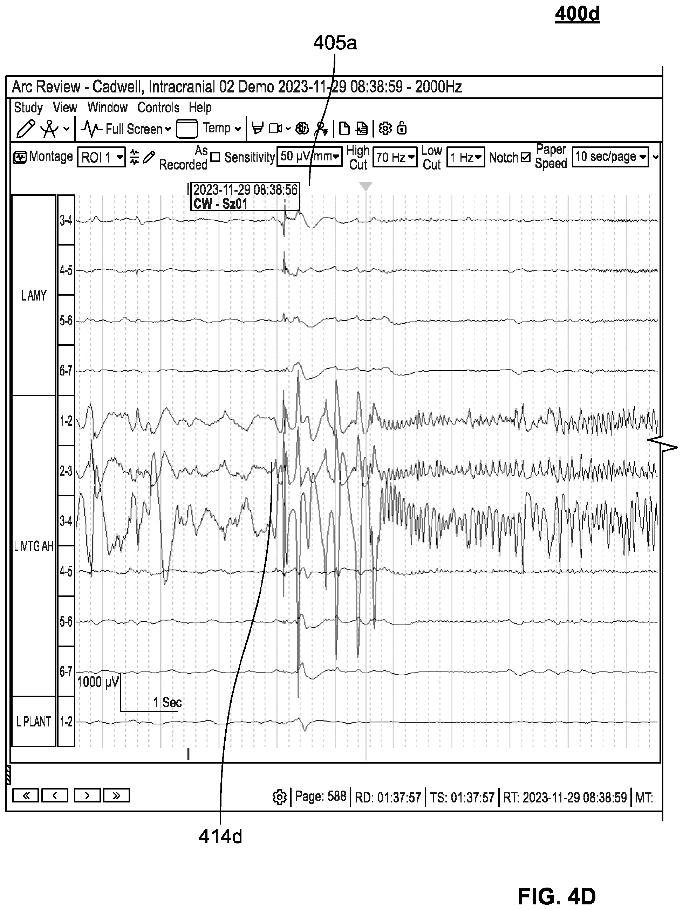

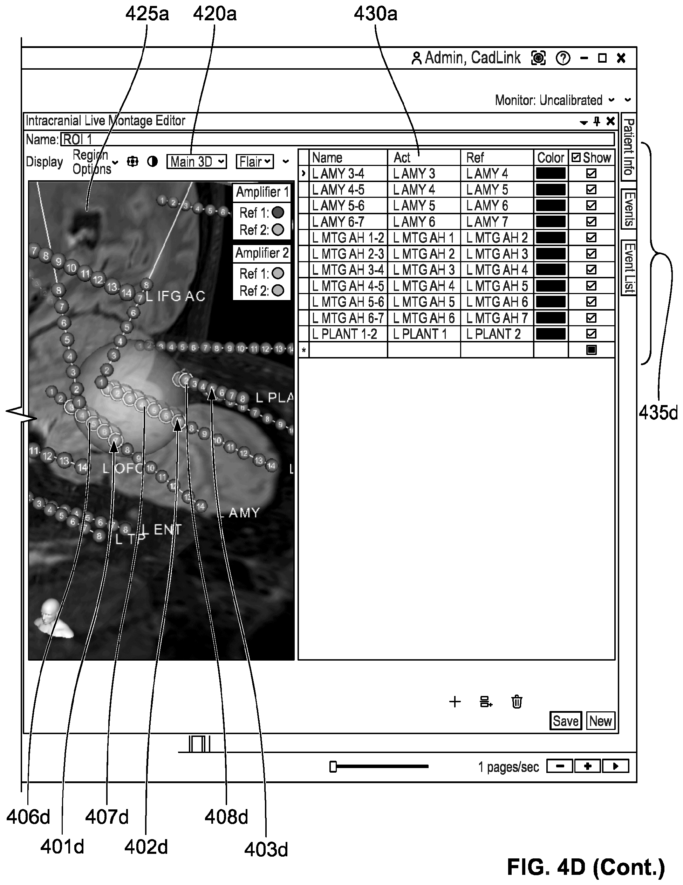

FIG. 4D illustrates a sixth unified viewing environment 400d that supports a clinician in automatically generating and displaying bipolar montages within specific electrodes that are associated with an anatomical region, in accordance with some embodiments of the present specification. In embodiments, the clinician selects contacts of interest across multiple electrodes within the 3D brain image to analyze targeted brain regions, with the system automating montage generation and visualization. In embodiments, the clinician selects a first set of one or more contacts of interest 406d within a first electrode 401d, a second set of one or more contacts of interest 407d within a second electrode 402d, and a third set of one or more contacts of interest 408d within a third electrode 403d in the 3D brain image 425a displayed in the third view area 420a. In some embodiments, the contacts of interest 406d, 407d, and 408d are associated with a specific anatomical or functional region of the brain. For example, contacts 406d may correspond to a specific brain region such as the hippocampus, contacts 407d may correspond to a connected functional area, such as the amygdala, and contacts 408d may correspond to a critical network node such as the thalamus. Activating the COI bipolar function triggers module 125 to create within-electrode bipolar pairs for each selected contact group. A list of the generated bipolar montages 435d is automatically displayed in the fourth view area 430a. Further, bipolar EEG traces 414d are automatically displayed, in some embodiments, in the first view area 405a. Alternatively, in some embodiments, the bipolar EEG traces 414d are automatically displayed in a separate view area such as, for example, in the fifth view area 431a shown in FIG. 4A.

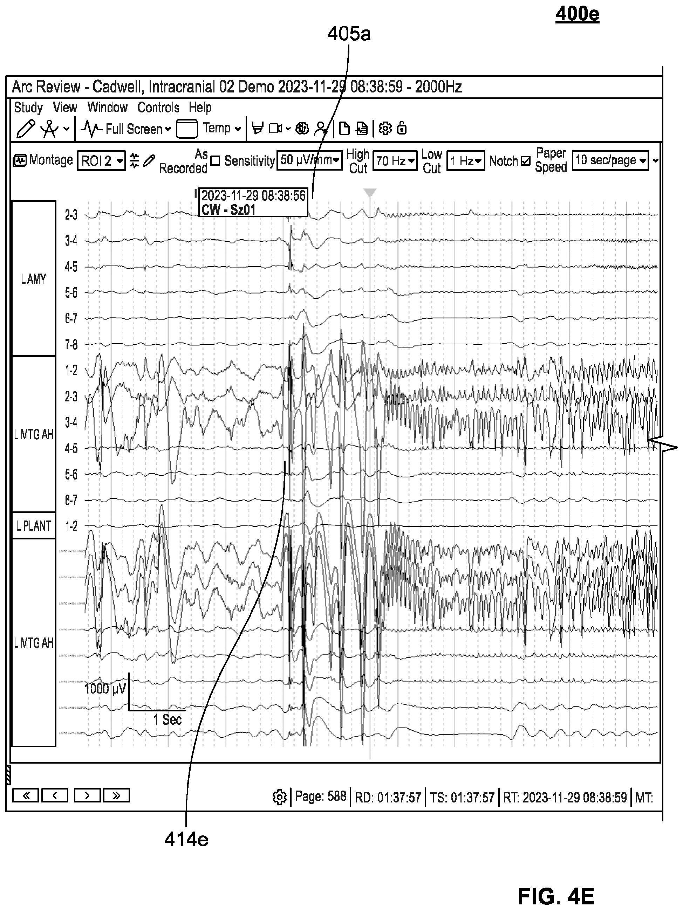

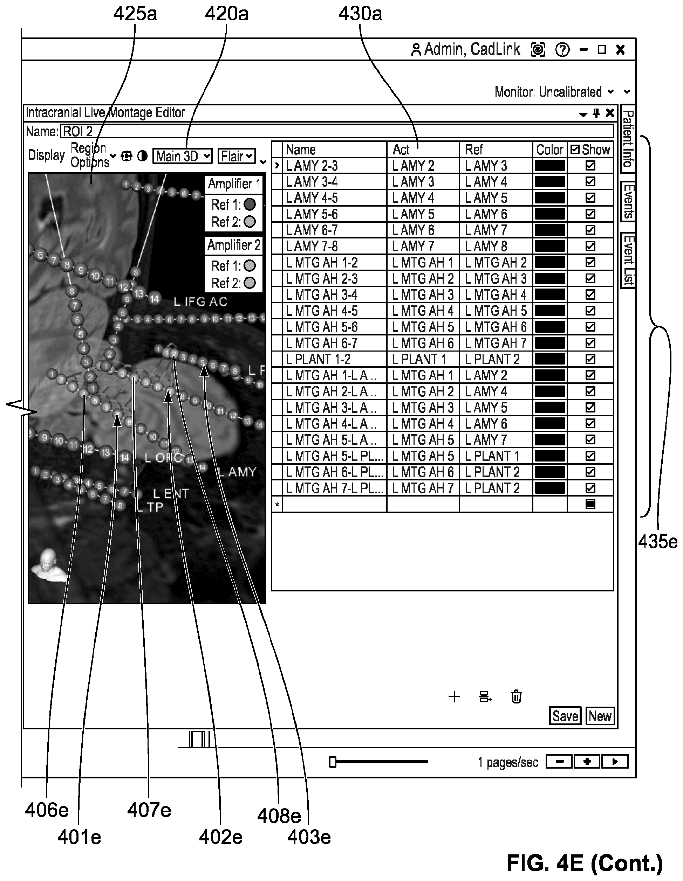

FIG. 4E illustrates a seventh unified viewing environment 400e that supports a clinician in automatically generating and displaying cross electrode bipolar montages associated with an anatomical region, in accordance with some embodiments of the present specification. As shown, in embodiments, the clinician selects a first set of one or more contacts of interest 406e within a first electrode 401e, a second set of one or more contacts of interest 407e within a second electrode 402e, and a third set of one or more contacts of interest 408e within a third electrode 403e in the 3D brain image 425a displayed in the third view area 420a. In some embodiments, the contacts of interest 406e, 407e, and 408e are associated with a specific anatomical or functional region of the brain. For example, contacts 406e may correspond to a specific brain region such as the hippocampus, contacts 407e may correspond to a connected functional area, such as the amygdala, and contacts 408e may correspond to a critical network node such as the thalamus. Activating the COI bipolar function triggers module 125 to create both within-electrode and across electrode bipolar pairs for each selected contact group. A list of the generated bipolar montages 435e is automatically displayed in the fourth view area 430a. Further, bipolar EEG traces 414e are automatically displayed, in some embodiments, in the first view area 405a. Alternatively, in some embodiments, the bipolar EEG traces 414e are automatically displayed in a separate view area such as, for example, in the fifth view area 431a shown in FIG. 4A.

### Referential Montages

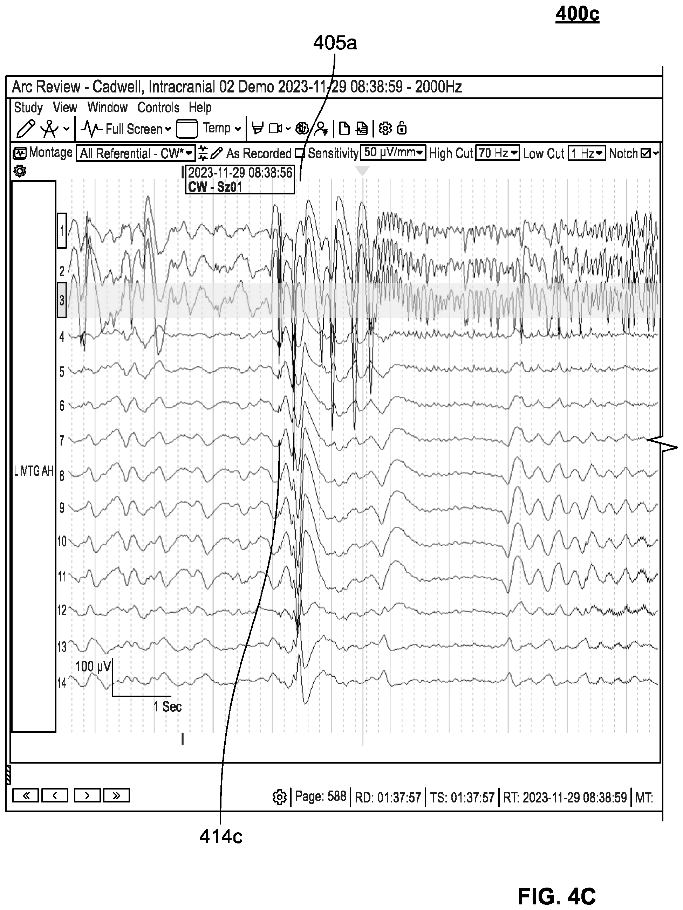

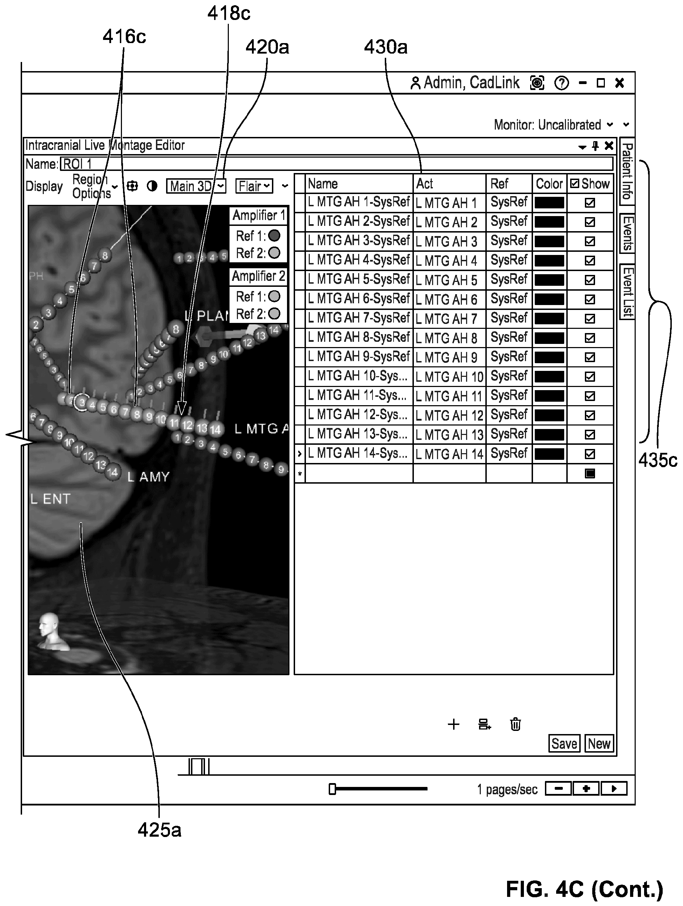

Module 125 enables clinicians to generate standard referential montages and provides the flexibility to dynamically choose any electrode as the reference. As used in the specification, standard refers to the current clinically used referential montages. These montages use what is commonly referred to as a system reference electrode as the reference electrode to be used for all other contacts. When a specific electrode is selected as the reference, all referential EEG traces are automatically updated to display activity relative to that chosen electrode, allowing for real-time adjustments and tailored analysis. FIG. 4C illustrates a fifth unified viewing environment 400c that supports a clinician in automatically generating and displaying a one or more referential montages in association with a visual representation of corresponding electrodes and contacts of interest within a patient's 3D brain image, in accordance with some embodiments of the present specification. As shown, the clinician may choose one or more contacts of interest 416c of an electrode 418c in the 3D brain image 425a displayed in the third view area 420a. When a clinician activates the COI referential montaging function, module 125 is configured to generate referential montages corresponding to the one or more contacts 416c. A list of the generated referential montages 435c is automatically displayed in the fourth view area 430a. Referential EEG traces 414c corresponding to the generated referential montages are automatically displayed, in some embodiments, in the first view area 405a, overlaying raw data for direct comparison. Alternatively, in some embodiments, the referential EEG traces 414c corresponding to the generated referential montages are automatically displayed in a separate view area such as, for example, in the fifth view area 431a shown in FIG. 4A.

Conventional, clinical practice is to define one static referential montage (typically using the system reference as the reference electrode). The dynamic reference electrode montage allows the user to point to any contact to be used as the reference contact and the corresponding referential montage is automatically updated displaying the resulting traces using this dynamic referential montage. For referential montaging, in some embodiments, the clinician can opt to dynamically display referential EEG traces 414c using one or more selected reference electrodes, enabling comparison and analysis of brain activity from different reference points. For example, if the patient has two SEEG electrodes (A, B) implanted with 4 contacts in each electrode (A1, A2, A3, A4 and B1, B2, B3, B4) the user can enable the dynamic referential montage mode and then point at contact B2, which would result in a montage with the following traces displayed (A1-B2, A2-B2, A3-B2, A4-B2, B1-B2, B3-B3, B4-B2). Pointing to another contact, for example A3, would result in the following traces to be displayed (A1-A3, A2-A3, A4-A3, B1-A3, B2-A3, B3-A3, and B4-A3).

### Averaged Reference Montages or Local White Matter Reference Montages

Average referencing is a referencing method where the electrical activity from all contacts within a defined spatial region, such as a sphere, cube, or custom-selected volume, is averaged and used as a reference for other contacts within that same region. Module 125 is configured to automatically determine which electrodes to include in the average based on their signal content or characteristics. If there is too much activity on an electrode, then that specific signal may be excluded from the average.

Module 125 is configured to use the 3D brain image to support EEG referencing accuracy by determining electrode positions relative to gray and white matter. In embodiments, module 125 is configured to calculate each electrode's distance to gray matter, which supports identification of contacts that are situated in or near white matter. The module 125 is also configured to automatically compute a white matter electrode reference by averaging activity from multiple white matter contacts within a defined spatial volume to mitigate noise. In some embodiments, the clinician may select a local white matter reference contact (for example, as the fifth parameter 470a in FIG. 4B) for each selected grey matter contact, thereby providing a potentially more accurate representation of the electrical activity generated at that specific electrode position.

### Dynamically Updated EEG Traces

Referring again to FIG. 4C, in some embodiments, module 125 is configured to enable real-time updates of referential EEG traces 414c, in a separate view area such as fifth view area 431a shown in FIG. 4A, as the clinician selects new contacts. In various embodiments, a reference contact can be selected by the clinician by a) moving, pointing or hovering the cursor over an EEG trace in the first view area 405a, b) moving, pointing or hovering the cursor over a contact in the 3D brain image 425a or c) selecting a contact manually from a displayed list of contacts (see list 335a of FIG. 3A). Upon selection or the contact or trace, the module 125 is configured to lock the reference contact and stop the dynamic updating of EEG traces 414c until a new COI montage is initiated. This allows the clinician to move through different contacts and see the corresponding referential EEG trace information related to the currently selected reference contact and the different trace display choices.

Referring back to FIG. 2, at step 218, based on the specified type of montage, the one or more contacts of interest identified and selected, and the one or more of the plurality of montage customization parameters activated in the previous steps, the module 125 generates and displays (in the at least one unified viewing environment) a group of one or more montages and associated EEG traces.

At step 220, based on the generated and displayed group of one or more montages and associated EEG traces, the clinician is supported in using the at least one unified viewing environment to activate and perform at least one of a plurality of predefined additional analyses such as, for example, frequency analysis and correlation. Since different types of montages are automatically available for use, the available derivations can be analyzed and only derivations with certain specific characteristics are displayed in the at least one unified viewing environment. The clinician can select the display of multiple different analyses and EEG traces at the same time.

It should be noted that the montages/derivations/traces that are used for analysis are the same traces that are displayed on the screen. The number of these traces are highly limited for a number of different reasons. EEG traces have traditionally been analyzed on paper with a limited number of traces being able to be drawn. The transition to a digital format has afforded the ability to view the traces simultaneously with their actual recordation using different montages. There is a limit, however, to the digital display screen space. The high number of traces (on the order of 100-250) in intracranial recordings are even more limited in how they can be displayed. However, in reality, when it comes to intracranial recordings there are many more traces that could be of interest to analyze (the mathematical number is the factorial of the number of contacts which would be in a range of 5*10^260) which is of course completely unmanageable. Therefore, the systems and methods of the present specification allow for the advantageous use of software algorithms intelligently selecting some (on the order of 1000) of the different derivations/traces and analyzing them in the background for different characteristics and displaying the accumulated results (for instance as 3D images) or selecting a smaller number (for instance 100) and displaying them as traces. The analysis of the many derivations may, for example, include using the neural networks for selection, cross-correlation between signals in time (analyzing how activity is spread in time), frequency analysis to detect High Frequency Oscillations (HFO), spike detection, seizure detection, low frequency detection, ML among others. Therefore, the systems and methods of the present specification do not limit the analysis to what is currently displayed on the screen but enable the analysis of a subset of all the other traces/derivations.

The above examples are merely illustrative of the many applications of the systems and methods of the present specification. Although only a few embodiments of the present invention have been described herein, it should be understood that the present invention might be embodied in many other specific forms without departing from the spirit or scope of the invention. Therefore, the present examples and embodiments are to be considered as illustrative and not restrictive, and the invention may be modified within the scope of the appended claims.

## Claims

1. A computer-implemented method of enabling a user to generate one or more montages using a computing device, wherein the computing device comprises at least one processor configured to execute programmatic instructions and a non-transient memory adapted to store the programmatic instructions, the method comprising:
generating a graphical user interface, wherein the graphical user interface comprises at least a first display area, a second display area and a third display area, wherein the first display area, the second display area and the third display area are concurrently displayed and visually separated from each other;
acquiring, using the computing device, an image of a patient's brain comprising data indicative of a visual representation of one or more electrodes placed in the patient's brain, wherein each of the one or more electrodes has associated one or more contacts therewith, and wherein the three-dimensional image is aligned with a three-dimensional coordinate system;
acquiring, using the computing device, EEG data of the patient, wherein the EEG data comprises a first plurality of EEG traces;
displaying, simultaneously, one or more of the first plurality of EEG traces in the first display area and the image in the second display area;
automatically visually associating the visual representation of each of the one or more electrodes and associated one or more contacts in the image with a corresponding one of the one or more first plurality of EEG traces;
receiving a user selection of one or more contacts in the second display area;
receiving a user selection of a first parameter provided in the third display area, wherein the first parameter is indicative of a type of montage, and wherein the third display area further comprises at least a second parameter and a third parameter for selection by the user; and
automatically generating said one or more montages based on the selected one or more contacts, the first parameter, and at least one of the second parameter or third parameter.

2. The computer-implemented method of claim 1, wherein the image is a three dimensional image of the patient's brain.

3. The computer-implemented method of claim 1 or 2, wherein the one or more contacts are selected from within an electrode of the one or more electrodes and/or from across the one or more electrodes.

4. The computer-implemented method of any preceding claim, wherein the type of montage is one of a bipolar montage, a referential montage or an average referential montage.

5. The computer-implemented method of any preceding claim, further comprising generating the one or more montages and displaying a second plurality of EEG traces corresponding to the generated one or more montages in the third display area.

6. The computer-implemented method of any preceding claim, further comprising:
receiving a user selection of a first contact of the one or more contacts; and
receiving a user selection of the second parameter, wherein the second parameter defines a neural network programmatically associated with an anatomical location of the first contact and wherein additional ones of the one or more contacts are selected automatically based on the selected neural network.

7. The computer-implemented method of any preceding claim, further comprising:
receiving a user selection of the third parameter, wherein the third parameter is indicative of at least one of an anatomical region or functional region of the patient's brain, wherein a portion of the one or more contacts are associated with at least one of the anatomical region or functional region, and wherein the one or more contacts are selected automatically based on the user selection of the third parameter.

8. The computer-implemented method of any preceding claim, wherein at least one of the one or more contacts is located in white matter, wherein at least one of the one or more contacts is positioned in gray matter, and wherein the computer-implemented further comprises receiving a user selection of a fourth parameter, wherein the fourth parameter is visually presented in the third display area and defines the at least one of the one or more contacts located in white matter as being a reference contact, and wherein the first parameter is defined as being a referential montage.

9. A computer-implemented method of enabling a user to generate one or more montages using a computing device, wherein the computing device comprises at least one processor configured to execute programmatic instructions and a non-transient memory adapted to store the programmatic instructions, the method comprising:
generating a graphical user interface, wherein the graphical user interface comprises at least a first display area, a second display area and a third display area, wherein each of the first display area, the second display area and the third display area is separate and concurrently displayed with each other;
acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is co-registered with a three-dimensional coordinate system;
acquiring, using the computing device, EEG data of the patient, wherein the EEG data comprises a first plurality of EEG traces;
displaying, simultaneously, at least a portion of the first plurality of EEG traces in the first display area and the post-surgical data in the second display area;
automatically visually associating the visual representation of the one or more electrodes and associated one or more contacts with a corresponding one of the first plurality of EEG traces;
receiving a user selection of a first contact of the one or more contacts in the second display area;
receiving a user selection of a first parameter displayed in the third display area, wherein the first parameter is indicative of a neural network;
automatically selecting a portion of the one or more contacts, wherein the portion of the one or more contacts is programmatically associated with the neural network;
receiving a user selection of a second parameter displayed in the third display area, wherein the second parameter is indicative of a type of montage; and
automatically generating the one or more montages based on the automatically selected one or more contacts of interest and the selected type of montage.

10. The computer-implemented method of claim 9, wherein the type of montage is one of a bipolar montage, a referential montage or an average referential montage.

11. The computer-implemented method of claim 9 or 10, further comprising displaying a second plurality of EEG traces corresponding to the generated one or more montages in the third display area.

12. A computer-implemented method of enabling a user to generate one or more montages using a computing device, wherein the computing device comprises at least one processor configured to execute programmatic instructions and a non-transient memory adapted to store the programmatic instructions, the method comprising:
generating a graphical user interface, wherein the graphical user interface comprises at least a first display area, a second display area and a third display area, wherein each of the first display area, the second display area and the third display area is separate and concurrently displayed with each other;
acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts;
acquiring, using the computing device, a first plurality of EEG traces of the patient;
displaying, simultaneously, at least a portion of the first plurality of EEG traces in the first display area and the post-surgical data in the second display area;
automatically visually associating the visual representation of each of the one or more electrodes and associated one or more contacts in the post-surgical data with a corresponding one of the first plurality of EEG traces;
receiving a user selection of a first parameter displayed in the third display area, wherein the first parameter is indicative of at least one of an anatomical region or functional region of the patient's brain;
automatically selecting a portion of the one or more contacts in the second display area, wherein the portion of the one or more contacts is associated with the selected anatomical region or functional region;
receiving a user selection of a second parameter displayed in the third display area, wherein the second parameter is indicative of a type of montage; and
automatically generating said one or more montages based on the automatically selected one or more contacts of interest and the selected second parameter.

13. The computer-implemented method of claim 12, wherein the type of montage is one of a bipolar montage, a referential montage or an average referential montage, wherein the three-dimensional image is co-registered with a three-dimensional coordinate system, and wherein the computer-implemented method further comprises displaying a second plurality of EEG traces corresponding to the generated one or more montages in the third display area.

14. A computer-implemented method of enabling a user to generate one or more montages using a computing device, wherein the computing device comprises at least one processor configured to execute programmatic instructions and a non-transient memory adapted to store the programmatic instructions, the method comprising:
generating a graphical user interface, wherein the graphical user interface comprises at least a first display area, a second display area and a third display area, wherein each of the first display area, the second display area and the third display area is visually separate when concurrently displayed;
acquiring, using the computing device, a three-dimensional image of a patient's brain comprising data indicative of a visual representation of one or more electrodes placed in the patient's brain, wherein each of the one or more electrodes has one or more contacts therewith, and wherein the three-dimensional image is co-registered with a three-dimensional coordinate system;
acquiring, using the computing device, a first plurality of EEG traces of the patient;
displaying, simultaneously, at least a portion of a the first plurality of EEG traces in the first display area and the post-surgical data in the second display area;
automatically visually associating the visual representation of at least a portion of the one or more contacts with a corresponding one of the first plurality of EEG traces;
receiving a user selection of the one or more contacts;
receiving, via the third display area, a user selection of a type of montage, wherein the type of montage is a referential montage;
receiving, via the third display area, a user selection of a second parameter, wherein the second parameter defines at least one of the one or more contacts as a reference contact; and
automatically generating said one or more montages based on the selected one or more contacts of interest, the type of montage and the reference contact.

15. The computer-implemented method of claim 14, wherein at least a portion of the selected one or more contacts is positioned in gray matter, wherein at least a portion of the selected one or more contacts is positioned in white matter, and wherein the computer-implemented method further comprises displaying the generated one or more montages in the second display area.
